(19) Europäisches Patentamt
European Patent Office
Office européen des brevets

(11) Veröffentlichungsnummer: **0 406 661 A1**

(12) **EUROPÄISCHE PATENTANMELDUNG**

(21) Anmeldenummer: **90112031.1**

(22) Anmeldetag: **25.06.90**

(51) Int. Cl.⁵: **C07D 271/04**, C07D 413/04, C07D 295/22, C07D 211/98, C07D 265/30, C07C 291/08, A61K 31/41, //(C07D413/04, 271:00,211:00),(C07D413/04, 271:00,265:00)

(30) Priorität: **30.06.89 DE 3921460**

(43) Veröffentlichungstag der Anmeldung: **09.01.91 Patentblatt 91/02**

(84) Benannte Vertragsstaaten: **AT BE CH DE DK ES FR GB IT LI NL SE**

(71) Anmelder: **CASSELLA Aktiengesellschaft**
**Hanauer Landstrasse 526**
**D-6000 Frankfurt am Main 61(DE)**

(72) Erfinder: **Kujath, Eckard, Dr.**
**Bonhoefferstrasse 27**
**D-6457 Maintal 1(DE)**
Erfinder: **Baumgartner, Christian, Dr.**
**Schwilkenhofstrasse 4**
**D-7000 Stuttgart 40(DE)**
Erfinder: **Schönafinger, Karl, Dr.**
**Holunderweg 8**
**D-8755 Alzenau(DE)**
Erfinder: **Beyerle, Rudi, Dr.**
**An der Pfaffenmauer 44**
**D-6000 Frankfurt/Main 60(DE)**
Erfinder: **Just, Melitta, Dr.**
**Theodor-Heuss-Strasse 80**
**D-6070 Langen(DE)**
Erfinder: **Bohn, Helmut, Dr.**
**Kranzbergring 11**
**D-6369 Schöneck 1(DE)**
Erfinder: **Ostrowski, Jörg, Dr.**
**Eschenstrasse 20**
**D-6458 Rodenbach(DE)**

(74) Vertreter: **Urbach, Hans-Georg, Dr. et al**
**Hanauer Landstrasse 526**
**D-6000 Frankfurt am Main 61(DE)**

(54) **Substituierte 3-Aminosydnonimine, Verfahren zu ihrer Herstellung und ihre Verwendung.**

(57) Substituierte 3-Aminosydnonimine der allgemeinen Formel I

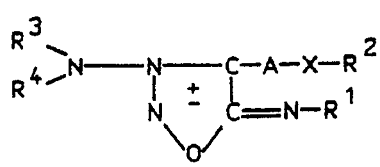

(I)

und ihre pharmakologisch annehmbaren Säureadditionsalze,
worin
A eine Alkylenkette;
X eine der Gruppen -O- oder -S- ;
$R^1$ z.B. Wasserstoff;
$R^2$ z.B. eine Alkylgruppe
bedeuten, werden durch Cyclisierung einer Verbindung der Formel II

EP 0 406 661 A1

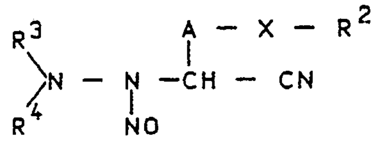

(II)

und gegebenenfalls anschließende Acylierung hergestellt und besitzen wertvolle pharmakologische Eigenschaften.

# SUBSTITUIERTE 3-AMINOSYDNONIMINE, VERFAHREN ZU IHRER HERSTELLUNG UND IHRE VERWENDUNG

Die Erfindung betrifft pharmakologisch wirksame substituierte 3-Aminosydnonimine der allgemeinen Formel I

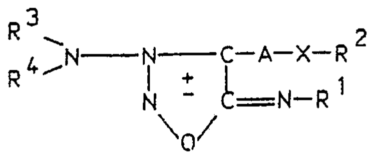

( I )

und ihre pharmakologisch annehmbaren Säureadditionsalze,
worin
A eine geradkettige oder verzweigte Alkylenkette mit 1 bis 6 C-Atomen;
X eine der Gruppen -O- oder -S- ;
$R^1$ Wasserstoff oder den Rest $-COR^5$;
$R^2$ eine Alkylgruppe mit 1 bis 6 C-Atomen, eine Alkenyl-oder Alkinylgruppe mit jeweils 3 bis 6 C-Atomen, eine gegebenenfalls substituierte Aralkylgruppe mit 1 bis 4 C-Atomen in der Alkyl- und 6 bis 10 C-Atomen in der Arylgruppe oder eine gegebenenfalls substituierte Arylgruppe mit 6 bis 10 C-Atomen;
$R^3$ und $R^4$ die gleich oder verschieden sein können, Alkylgruppen mit 1 bis 6 C-Atomen, Alkenyl-, Alkinyl- oder Cycloalkylgruppen mit jeweils 3 bis 6 C-Atomen, gegebenenfalls substituierte Aralkylgruppen mit 1 bis 4 C-Atomen in der Alkylgruppe und 6 bis 10 C-Atomen in der Arylgruppe, gegebenenfalls substituierte Arylgruppen mit 6 bis 10 C-Atomen, heterocyclische Gruppen mit 3 bis 5 Ring-C-Atomen und der Gruppe $-S(O)_m-$ oder $R^3$ und $R^4$ zusammen mit dem Stickstoffatom, an das sie gebunden sind, einen heterocyclischen Ring der Formel

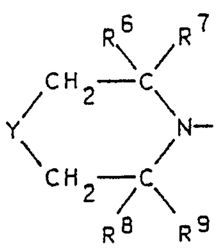

;

$R^5$ einen aliphatischen Rest mit 1 bis 6 C-Atomen, der auch durch Alkoxy mit 1 bis 6 C-Atomen oder durch einen aliphatischen Thiorest mit bis zu 4 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen, der auch durch Alkoxy mit 1 bis 6 C-Atomen substituiert sein kann; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 4 C-Atomen und/oder 1 bis 3 Alkoxyreste mit 1 bis 3 C-Atomen und/oder 1 oder 2 Nitrogruppen und/oder 1 oder 2 Hydroxygruppen und/oder 1 oder 2 Alkylcarbonyloxyreste mit 1 bis 4 C-Atomen und/oder 1 bis 3 Alkylthioreste mit 1 bis 4-C-Atomen und/oder einen Trifluormethylrest und/oder einen Imidazolylrest mono-, di- oder trisubstituierten Arylrest mit 6 bis 10 C-Atomen; Imidazolyl; Pyridyl; Thienyl; Styryl;
m eine der Zahlen 0, 1 oder 2;
Y eine der Gruppen $-(CH_2)_n-$, -O-, $-S(O)_n-$ oder $-N(R^{10})-$;
$R^6$, $R^7$, $R^8$, und $R^9$, die gleich oder verschieden sein können, Wasserstoff oder Alkylgruppen mit 1 bis 4 C-Atomen;
n eine der Zahlen 0, 1 oder 2;
$R^{10}$ einen Alkylrest mit 1 bis 4 C-Atomen, einen gegebenenfalls substituierten Arylrest mit 6 bis 12 C-Atomen oder einen Rest der Formel $-COOR^{11}$, -COH, $-COR^{11}$ oder $-S(O)_2R^{11}$;
$R^{11}$ einen Alkylrest mit 1 bis 4 C-Atomen

bedeuten.

Die Erfindung betrifft ferner ein Verfahren zur Herstellung der erfindungsgemäßen Verbindungen I und ihre Verwendung.

Aliphatische Reste, Thioreste, Alkylreste, Alkoxyreste, Alkenylreste, Alkinylreste, Alkoxyreste können geradkettig oder verzweigt sein. Dies gilt auch, wenn sie als Substituenten anderer Reste, z.B. als Substituenten für Arylreste, oder in Verbindung mit anderen Resten auftreten, z.B. als Aralkyl, als Alkoxycarbonyl, als Alkylcarbonyloxy oder als Alkoxyalkoxy.

Arylreste können unsubstituiert oder durch 1 bis 3 Halogenatome und/oder 1 bis 3 Alkylreste mit 1 bis 4 C-Atomen und/oder 1 bis 3 Alkoxyreste mit 1 bis 4 C-Atomen und/oder 1 bis 2 Nitrogruppen und/oder 1 bis 3 Hydroxygruppen und/oder 1 bis 3 Alkylthioreste mit 1 bis 4 C-Atomen und/oder einen Trifluormethylrest mono-, di- oder trisubstituiert sein. Dies gilt auch, wenn sie als Substituenten anderer Reste, z.B. als Aralkyl, auftreten.

Beispiele für A sind die Methylengruppe, die Ethylengruppe, die Trimethylengruppe, die Tetramethylengruppe, die durch eine Methylgruppe, eine Ethylgruppe oder eine Propylgruppe substituierte Methylengruppe, die durch eine, zwei oder drei Methylgruppen substituierte Ethylengruppe oder die durch eine oder zwei Ethylgruppen substituierte Ethylengruppe. Für A sind die Methylengruppe und die 1-Methyl-ethylengruppe besonders bevorzugt.

Beispiele für $R^2$ sind die Methyl-, die Ethyl-, die Propyl-, die Butyl-, die Hexyl-, die Isopropyl-, die sec-Butyl-, die Isobutyl-, die 2-Propenyl-, die 2-Butenyl-, die 3-Butenyl-, die 3-Methyl-2-butenyl-, die 2,3-Dimethyl-2-butenyl, die 4-Methyl-3-pentenyl-, die 2-Propinyl-, die 2-Butinyl-, die 2-Pentinyl-, die 4-Methyl-2-pentinyl-, die Benzyl-, die 2-Phenethyl-, die 3-Phenylpropyl, die 3-Phenylbutyl, die 1-Naphthylmethyl-, die 2-Naphthylmethyl, die Chlorbenzyl-, die Methylbenzyl-, die Methoxybenzyl-, die Ethoxybenzyl-, die Dimethoxybenzyl-, die Methylthiobenzyl-, die Trifluor methylbenzyl-, die Phenyl-, die 1-Naphthyl-, die 2-Naphthyl-, die Chlorphenyl-, die Dichlorphenyl-, die Fluorphenyl-, die Tolyl-, die Methoxyphenyl-, die Ethoxyphenyl- oder die Trifluormethylphenylgruppe. Bevorzugt für $R^2$ sind Alkylgruppen mit 1 bis 6 C-Atomen, Alkenyl-und Alkinylgruppen mit jeweils 3 bis 5 C-Atomen, Aralkylgruppen mit 1 bis 3 C-Atomen in der Alkylgruppe und einem unsubstituierten oder mono- oder disubstituierten Phenylrest als Arylgruppe und Arylgruppen, die einen unsubstituierten, mono- oder disubstituierten Phenylrest darstellen. Für $R^2$ sind besonders bevorzugt: Ethyl, Phenyl, Benzyl und Allyl.

Beispiele für die sekundäre Aminogruppe, die durch $R^3$ und $R^4$ und das Stickstoffatom, an das diese Reste gebunden sind, gebildet wird, sind die Dimethylamino-, die Diethylamino-, die Di-n-butylamino-, die Diisobutylamino-, die Dihexylamino-, die tert. -Butyl-methylamino-, die Di-2-propenylamino-, die Di-2-propinylamino-, die Methyl-2-propinylamino-, die Dicyclohexylamino-, die Cyclohexylmethylamino-, die Dibenzylamino-, die Diphenethylamino-, die Benzylmethylamino-, die Diphenylamino-, die Methylphenylamino-, die Benzylphenylamino-, die (1,1-Dioxo-tetrahydrothiophen-3-yl)methylamino-gruppe. Beispiele für die Gruppe des heterocyclischen Ringes, die $R^3$ und $R^4$ zusammen mit dem Stickstoffatom, an das sie gebunden sind, bilden können, sind: die Pyrrolidino-, die 2,5-Dimethylpyrrolidino, die 2,2,5,5-Tetramethylpyrrolidino-, die Piperidino-, die 2-Methylpiperidino-, die 2,2-Dimethylpiperidino-, die 2,6-Dimethylpiperidino-, die 2,2,6,6-Tetramethylpiperidino-, die Perhydroazepin-1-yl-, die Morpholino-, die 3,3-Dimethylmorpholino-, die 3,3,5,5-Tetramethylmorpholino-, die Tetrahydro-1,4-thiazin-4-yl-, die 1,1-Dioxotetrahydro-1,4-thiazin-4-yl-, die 4-Methyl-1-piperazinyl-, die 4-Propyl-1-piperazinyl-, die 4-Phenyl-1-piperazinyl-, die 4-(2-Methoxyphenyl)-1-pipe razinyl-, die 4-(Methoxycarbonyl)-1-piperazinyl, die 4-(Ethoxycarbonyl)-1-piperazinyl-, die 4-Formyl-1-piperazinyl-, die 4-Acetyl-1-piperazinyl- oder die 4-Methansulfonyl-1-piperazinylgruppe. $R^3$ und $R^4$ bilden vorzugsweise zusammen mit dem Stickstoffatom, an das sie gebunden sind, einen heterocyclischen Ring der Formel

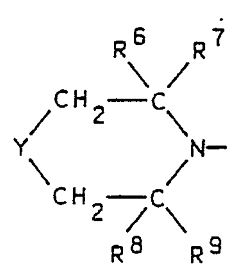

wobei Y eine der Gruppen $-(CH_2)_o-$, $-(CH_2)_1-$, -O-, -S-, $-S(O)_2-$ bedeutet und $R^6$, $R^7$, $R^8$ (d.h. eine direkte Bindung), und $R^9$, die gleich oder verschieden sein können, Wasserstoff oder Alkylreste mit 1 bis 4 C-Atomen, besonders bevorzugt Wasserstoff oder Methylgruppen, bedeuten. Beispiele für besonders bevorzugte heterocyclische Ringe sind der Pyrrolidino-, der 2,5-Dimethylpyrrolidino-, der Piperidino-, der 2,2-Dimethylpiperidino-, der 2,6-Dimethylpiperidino-, der 2,2,6,6-Tetramethylpiperidino-, der Morpholino-, der 3,3-Dimethylmorpholino-, der 3,3,5,5-Tetramethylmorpholino-, der Tetrahydro-1,4-thiazin-4-yl-und der 1,1-Dioxotetrahydro-1,4-thiazin-4-ylrest. Ganz besonders bevorzugt sind der 2,6-Dimethylpiperidino- und der Morpholino-rest.

Als für $R^5$ stehende aliphatische Reste kommen insbesondere Alkylreste, vorzugsweise mit 1 bis 4 C-Atomen, in Betracht. Die für $R^5$ stehenden aliphatischen Reste, insbesondere Alkylreste, können auch mit Alkoxy mit 1 bis 6 C-Atomen, insbesondere 1 bis 4 C-Atomen, vorzugsweise 1 bis 3 C-Atomen, substituiert sein. Beispiele für Alkyl- und Alkoxyalkylreste, die für $R^5$ stehen können, sind: Methyl; Ethyl; n-Propyl; i-Propyl; n-, i-, sec.- und tert.-Butyl; n- und i-Pentyl; n- und i-Hexyl; Methoxy-, Ethoxy-, n-Propoxy-, i-Propoxy-, n-Butoxy-, i-Butoxymethyl; 2-Methoxy-, 2-Ethoxy-, 2-n-Propoxy-, 2-i-Propoxy-, 2-n-Butoxy-ethyl; 2-Methoxy-, 3-Ethoxy-, 3-n-Propoxy-, 3-i-Propoxy-n-propyl oder -i-propyl. Die für $R^5$ stehenden aliphatischen Reste, insbesondere die Alkylreste, können auch mit einem aliphatisch substituierten Thiorest mit bis zu 4 C-Atomen substituiert sein. Derartige aliphatische Thioreste sind zum Beispiel Alkylthioreste mit 1 bis 4 C-Atomen, wie zum Beispiel Methyl-, Ethyl-, n-Propyl-, i-Propyl-, n-Butyl-, tert.-Butyl-thio, vorzugsweise aber Allylthio ($CH_2=CH\text{-}CH_2\text{-}S\text{-}$). Als für $R^1$ stehende cycloaliphatische Reste kommen vor allem Cycloalkylreste mit 5 bis 7 C-Atomen, insbesondere Cyclopentyl, und bevorzugt Cyclohexyl, in Betracht. Als für $R^5$ stehender bicycloaliphatischer Rest kommt insbesondere das 2,6,6-Trimethylbicyclo(3.1.1)heptan-3-yl (=Pinanyl-3) in Betracht. Als für $R^5$ stehender tricycloaliphatischer Rest kommt insbesondere das Tricyclo-$(3.3.1.1^{3,7})$decan-1-yl (=Adamant-1-yl) in Betracht.

Die Alkoxysubstituenten für die Alkoxyreste besitzen insbesondere 1 bis 4 C-Atome. Beispiele für Alkoxyreste und Alkoxyalkoxyreste, die für $R^5$ stehen können sind: Methoxy; Ethoxy; n- und i-Propoxy; n-, i-, sec.- und tert.-Butoxy; n-Pentyloxy; i-Hexyloxy; n-Octyloxy; n-Dodecyloxy; n-Hexadecyloxy; n-Heptadecyloxy; n-Octadecyloxy; Methoxy-, Ethoxy-, n-Propoxy-, i-Propoxy-, n-Butoxy-methoxy; 2-Methoxy-, 2-Ethoxy-, 2-n-Propoxy-, 2-i-Propoxy-ethoxy; 3-Methoxy-, 3-Ethoxy-, 3-n-Propoxy-, 3-i-Propoxy-propoxy; 4-Methoxy-, 4-Ethoxy-, 4-n-Propoxy-, 3-Propoxy-, 4-n-Butoxy-butoxy.

Der für $R^5$ stehende Alkoxycarbonylrest besitzt vorzugsweise 2 bis 5 C-Atome. Beispielsweise seien hierfür ge nannt: Methoxy-, Ethoxy-, n-Propoxy-, i-Propoxy-, n-Butoxy-, i-Butoxy-carbonyl. Als für $R^5$ stehender Alkoxycarbonylrest kommt insbesondere der Ethoxycarbonylrest in Betracht.

Als für $R^5$ stehende substituierte und unsubstituierte Arylreste sind z.B. α- oder β-Naphthylreste, insbesondere aber der Phenylrest, zu nennen. Als für $R^5$ stehende Aryloxyreste sind z.B. α- oder β-Naphthoxyreste, insbesondere aber der Phenoxyrest, zu nennen. Die für $R^5$ stehenden Arylreste können mono-, di- oder trisubstituiert sein, wobei jedoch auch bei einer Trisubstitution nur maximal 2 Nitrogruppen vorhanden sein können, wie beispielsweise 2-Methyl-4,6-dinitrophenyl und 2-Chlor-6-methyl-4-nitrophenyl. Bei sperrigen Substituenten ist evtl. nur eine Di- oder Monosubstitution möglich. Als Halogensubstituenten für die Arylreste kommen z.B. Fluor-, Chlor- und/oder Bromatome in Betracht. Als Alkylcarbonyloxysubstituenten für die Arylreste, insbesondere für einen Phenylrest, sind z.B. zu nennen: Acetoxy, n-Propionyloxy, i-Propionyloxy, n-Butyryloxy, i-Butyryloxy.

Beispiele für die für $R^5$ stehenden, gegebenenfalls substituierten Arylreste sind: Phenyl, 2-, 3- oder 4-Methyl-, -Ethyl-, -n-Propyl-, -i-Propyl-phenyl; 2-, 3-oder 4-Methoxy-, -Ethoxy-, -n-Propoxy-, -i-Propoxyphenyl; 2-, 3- oder 4-Fluor-, -Chlor- oder -Brom-phenyl; 2-, 3- oder 4-Nitrophenyl; 2-, 3- oder 4-Hydroxyphenyl; 2-, 3- oder 4-Acetoxy-, -n-Propionyloxy-, -n-Butyryloxyphenyl; 2,3-, 2,4-, 2,5- oder 2,6-Dimethyl-, -Diethyl-, -Dipropyl-phenyl; 2- oder 3-Methyl-4-chlorphenyl; 2- oder 3-Ethyl-4-fluorphenyl; 2-Chlor-4-ethylphenyl; 2-Brom-4-i-propylphenyl; 2,6-Diethoxy-4-chlorphenyl; 2,3,4-, 3,4,5-oder 2,3,5-Trimethyl-, -Triethyl-, -Tripropyl-, -Trimethoxy-, -Triethoxy- oder Tripropoxy-phenyl; 2-Hydroxy-3-, -4- oder -5-chlorphenyl; 2-Methyl-3-, -4- oder -5-acetoxy-phenyl.

Als für $R^5$ stehende substituierte Arylreste sind insbesondere zu nennen: Methylphenyl (= Tolyl), Methoxyphenyl und Chlorphenyl. Der für $R^5$ stehende Imidazolylrest ist vorzugsweise ein 1-Imidazolylrest.

Für $R^5$ sind bevorzugt: Methyl, Ethyl, Cyclohexyl, Phenyl, 4-Chlorphenyl, 4-Methoxyphenyl, Methoxy, Ethoxy, n-Propoxy, i-Propoxy, n-Decyloxy, n-Octadecyloxy, 2-n-Propoxyethoxy, 2-i-Propoxy-ethoxy, n-Butoxymethyl, 2-n-Butoxyethoxy und Allylthiomethyl. Für $R^5$ ganz besonders bevorzugt sind Ethoxy, Phenyl und Methoxyphenyl, insbesondere 4-Methoxyphenyl.

Die für $R^{10}$ stehenden gegebenenfalls substituierten Arylreste mit 6 - 12 C-Atomen können beispielsweise Phenyl-, 1-Naphthyl-, 2-Naphthyl- oder Biphenylyl-Reste sein.

Bevorzugte Verbindungen der Formel I sind solche, die einen oder insbesondere mehrere der bevorzugten, vor allem der besonders bevorzugten Reste enthalten. Ganz besonders bevorzugte erfindungsgemäße Verbindungen sind das 4-Benzylthiomethyl-3-(2,6-dimethylpiperidino)-sydnonimin und das 3-(2,6-Dimethylpiperidino)-4-phenoxy-methylsydnonimin und ihre pharmakologisch annehmbaren Säureadditionssalze, insbesondere ihre Hydrochloride.

Eine Verbindung der allgemeinen Formel I kann dadurch hergestellt werden, daß eine substituierte N-Nitrosoaminoacetonitril-Verbindung der allgemeinen Formel II

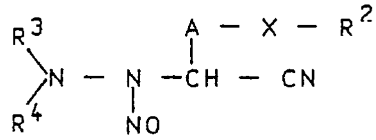

(II)

worin A, X, $R^2$, $R^3$ und $R^4$ die bereits genannten Bedeutungen besitzen, zu einer Verbindung der allgemeinen Formel Ia

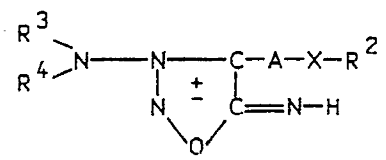

(Ia)

cyclisiert wird und daß diese oder ein Säureadditionssalz davon für den Fall, daß eine Verbindung der Formel I mit $R^1$ = $-COR^5$ hergestellt werden soll, mit einem Acylierungsmittel, das den Rest $-COR^5$ einführt, acyliert wird und die so erhaltene Verbindung gegebenenfalls in ein pharmakologisch annehmbares Säureadditionssalz überführt wird.

Die Cyclisierung der Verbindung II zu der Verbindung Ia wird in einem geeigneten organischen oder anorganischen Lösungs-, Dispergier- oder Verdünnungsmittel unter Zusatz eines Cyclisierungsmittels, normalerweise bei Temperaturen von -10 bis 40°C, insbesondere 0 bis 40°C, vorzugsweise bei 0 bis 20°C, durchgeführt.

Als Cyclisierungsmittel sind solche geeignet, die in wäßriger Lösung einen pH-Wert von 3 oder darunter einstellen, also z.B. starke Säuren, wie Mineralsäuren, wie Schwefel-, Salpeter- oder Phosphorsäure, vorzugsweise Chlorwasserstoff, aber auch starke organische Säuren, wie Sulfonsäuren oder Trifluoressigsäure. Die Cyclisierung wird normalerweise unter Eiskühlung durchgeführt. Von dem Cyclisierungsmittel kommen z.B. bezogen auf 1 mol der Verbindung der Formel II 0,1 bis 10 mol, vorzugsweise 1 bis 5 mol, zur Anwendung. Das Cyclisierungsmittel wird normalerweise im Überschuß eingesetzt. Besonders bequem ist die Verwendung von Chlorwasserstoff -als Cyclisierungsmittel, der normalerweise bis zur Sättigung in den Reaktionsansatz eingeleitet wird. Bei der Cyclisierung wird normalerweise das entsprechende Säureadditionssalz der Verbindung Ia erhalten.

Geeignete Lösungs-, Dispergier- oder Verdünnungsmittel sind z.B. : Alkohole, beispielsweise solche mit 1 bis 8 C-Atomen, insbesondere solche mit 1 bis 6 C-Atomen, vorzugsweise solche mit 1 bis 4 C-Atomen, wie z.B. Methanol, Ethanol, i- und n-Propanol, i-, sec- und tert-Butanol, n-, i-, sec-, tert-Pentanol, n-Hexanol, 2-Ethylbutanol, 2-Ethylhexanol, Isooctylalkohol, Cyclopentanol, Cyclohexanol, Methylcyclohexanol (Gemisch), Benzylalkohol; Ether, insbesondere solche mit 2 bis 8 C-Atomen im Molekül, wie z.B. Diethylether, Methyl-ethylether, Di-n-propyl-ether, Di-isopropyl-ether, Methyl-n-butylether, Methyl-tert-butylether, Ethyl-propyl-ether, Di-butyl-ether, Tetrahydrofuran, 1,4-Dioxan, 1,2-Dimethoxyethan, Bis-β-methoxyethylether; Oligoethylen-glykol-dimethyl-ether, wie z.B. Tetraglyme oder Pentaglyme; Carbonsäurealkylester, insbesondere solche mit 2 bis 10 C-Atomen im Molekül, wie z.B. Ameisensäure-methyl-, -ethyl-, -butyl- oder -isobutyl-ester, Essigsäure-methyl-, -ethyl-, -propyl-, isopropyl-, -butyl-, -isobutyl- oder -sec-butyl-, -amyl-, -isoamyl-, -hexyl-, -cyclohexyl- oder -benzyl-ester, Propionsäure-methyl-, -ethyl- oder -butyl-ester; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Methylcyclohexanon, Dimethylcyclohexanon, Benzophenon, Acetophenon; aliphatische Kohlenwasserstoffe, wie z.B. Hexan, Heptan, niedrig-und hochsiedende Petrolether, Spezialbenzine und Testbenzin; cycloaliphatische Kohlenwasserstoffe, wie z.B. Cyclopentan, Cyclohexan, Methylcyclohexan, Tetralin, Decalin; aromatische Kohlenwasserstoffe, wie z.B. Benzol, Toluol, o-, m- und p-Xylol, Ethylbenzol; halogenierte aliphatische oder aromatische Kohlenwasserstoffe, wie z.B. Methylenchlorid, Chloroform, Tetrachlorkohlenstoff, 1,2-Dichlorethan, Chlorbenzol, Dichlorbenzol; Hexamethylphosphorsäuretriamid; Sulfoxide, wie z.B. Dimethyl-sulfoxid; Tetramethylensulfon; Wasser. Auch Gemische verschiedener Lösungs- oder Dispergiermittel können verwendet werden, beispielsweise Wasser-Methanol oder vorzugsweise Essigsäureethylester-Methanol.

Die Verbindungen der Formel Ia stellen erfindungsgemäße Verbindungen der allgemeinen Formel I für den Fall dar, daß $R^1$ = Wasserstoff ist.

Die Acylierung der Verbindung der Formel Ia, die auch in Form eines Säureadditionssalzes vorliegen kann, zur Einführung des Restes $R^1$ = $-COR^5$ kann in an sich bekannter Weise mit einem geeigneten Acylierungsmittel der Formel III durchgeführt werden

$$X-\overset{\overset{\displaystyle O}{\|}}{C}-R^5 \qquad (III),$$

worin X einen nukleophil abspaltbaren Rest darstellt.

In der Formel III bedeutet X z.B. insbesondere Halogen, vorzugsweise -Cl oder -Br; -OH; -O-Alkyl, insbesondere mit 1 bis 5 C-Atomen; -O-Aryl, wobei der Arylrest insbesondere ein Phenylrest ist, der auch mit Alkyl, insbesondere Methyl, und/oder Nitro ein- oder mehrfach substituiert sein kann und beispielsweise ein Tolyl-, Dinitrophenyl- oder Nitrophenyl-Rest ist; $-O-CO-R^5$; -O-CO-O-Alkyl, insbesondere mit 1 bis 5 C-Atomen im Alkylrest, oder den über ein N-Atom gebundenen Rest eines Azols oder Benzazols mit mindestens 2 N-Atomen im quasi-aromatischen Fünfring.

Die Acylierung wird zweckmäßigerweise in flüssiger Phase in Anwesenheit eines inerten Lösungs-, Dispergier- oder Verdünnungsmittels oder in einem Überschuß des Acylierungsmittels, zweckmäßigerweise unter Rühren, durchgeführt.

Bei der Acylierung wird das Acylierungsmittel der Formel III zweckmäßigerweise in äquivalenter Menge oder in einem geringen molaren Überschuß eingesetzt. Überschüsse von bis zu 30 mol% sind in der Regel ausreichend, d.h. das Molverhältnis zwischen der Verbindung der Formel Ia und dem Acylierungsmittel der Formel III beträgt zweckmäßigerweise 1 : (1 bis 1,3), vorzugsweise 1: (1 bis 1,2). Falls bei der Acylierungsreaktion eine Säure abgespalten wird, ist der Zusatz eines Säurefängers, wie z.B. eines Alkalihydroxids, wie z.B. Natrium-, Kalium-oder Lithiumhydroxid, eines tertiären organischen Amins, wie z.B. Pyridin oder Triethylamin, eines Alkalicarbonats oder Alkalibicarbonats, wie z.B. Soda oder Natriumbicarbonat, oder eines Alkalisalzes einer schwachen organischen Säure, wie z.B. Natriumacetat, zweckmäßig. Bei der Acylierungsreaktion können auch geeignete Katalysatoren, wie z.B. 4-Dimethylaminopyridin, zugesetzt werden.

Die Acylierung kann prinzipiell bei Temperaturen zwischen -10 °C und dem Siedepunkt des verwendeten Lösungs-, Dispergier- oder Verdünnungsmittels erfolgen. In vielen Fällen wird die Umsetzung bei 0 bis 50°C, insbesondere bei 0 bis 30°C und vorzugsweise bei Raumtemperatur, durchgeführt.

Die Verbindungen der Formel III sind Acylierungsmittel und stellen somit z.B. dar: für X = Halogen: Säurehalogenide bzw. Halogenameisensäureester, von denen Säurechloride und Chlorameisensäureester bevorzugt sind; für -OH: Carbonsäuren; für -O-Alkyl und -O-Aryl: Ester, von denen die Tolyl-, 2,4-Dinitro- oder 4-Nitrophenylester bevorzugt sind; für $-O-CO-R^5$: Anhydride; für -O-CO-O-Alkyl: gemischte Carbonsäure-Kohlensäure-Anhydride; oder heterocyclische Amide oder Azolide. Die Acylierungsmittel der Formel III können nach an sich bekannten Verfahren hergestellt werden.

Bei der Verwendung einer Carbonsäure als Acylierungsmittel ist der Zusatz eines Aktivierungsmittels zweckmäßig, das die Aufgabe hat, das Acylierungspotential der Carbonsäure zu erhöhen oder zu aktivieren bzw. die Carbonsäure in situ oder vorzugsweise kurz vor der Umsetzung mit der Verbindung der Formel Ia in ein reaktives Carbonsäurederivat der Formel III zu überführen. Als derartige Aktivierungsmittel sind z.B. geeignet: N,N′-disubstituierte Carbodiimide, insbesondere wenn sie mindestens einen sekundären oder tertiären Alkylrest enthalten, wie z.B. Diisopropyl-, Dicyclohexyl- oder N-Methyl-N′-tert.butyl-carbodiimid (vgl. Methodicum Chimicum, Verlag G.Thieme, Stuttgart, Bd. 6, (1974), S.682/683, und Houben-Weyl, Methoden der Org. Chemie, Bd.8, (1952), S. 521/522); Kohlensäurederivate, wie z.B. Phosgen, Chlorameisensäureester, insbesondere mit 1 bis 5 C-Atomen im Alkylrest (vgl. z.B. Tetrahedron Letters 24 (1983), 3365 bis 3368); Kohlensäureester, wie z.B. N,N′-Disuccinimido-carbonat, Diphthalimido-carbonat, 1,1′-(Carbonyldioxy)-dibenzo-triazol oder Di-2-pyridyl-carbonat (vgl. z.B. Tetrahedron Letters, Vol.25, No.43, 4943-4946), gegebenenfalls in Anwesenheit geeigneter Katalysatoren, wie z.B. 4-Dimethylaminopyridin. Ferner sind als Aktivierungsmittel N,N′-Carbonyldiazole, wie z.B. N,N′-Carbonyl-diimidazol, 2,2′-Carbonyl-ditriazol(1,2,3), 1,1′-Carbonyl-ditriazol(1,2,4), N,N′-Carbonyl-dipyrazol, 2,2′-Carbonyl-ditetrazol, N,N′-Carbonyl-benzimidazol oder N,N′-Carbonylbenztriazol, geeignet (vgl. z.B. H.A. Staab, M. Lücking und F.H.Dürr, Chem. Ber. 95 , (1962), 1275 ff, H. A. Staab und A. Mannschreck, Chem. Ber. 95 , (1962), 1284 ff.; H.A. Staab und W. Rohr, "Synthesen mit heterocyclischen Amiden (Azoliden)" in "Neuere Methoden der Präparativen Organischen Chemie, Band V, Verlag Chemie, 1967, S. 53 ff., insbesondere S. 65 bis 69). Als

N,N′-Carbonyl-diazol wird häufig das käufliche N,N′-Carbonyl-diimidazol verwendet. Die anderen N,N′-Carbonylazole sind aber aus dem jeweiligen Azol und Phosgen ebenfalls leicht zugänglich.

Als Aktivierungsmittel für Carbonsäuren sind ferner geeignet: Derivate der Oxalsäure, wie z.B. Oxalylchlorid (vgl. z.B. GB-PS 2 139 225) oder N,N′-Oxalyl-diazole wie z.B. 1,1 ,-Oxalyldi-imidazol, 1,1′-Oxalyldi-1,2,4-triazol und 1,1′-Oxalyldi-1,2,3,4-tetrazol (vgl. z.B. Shizuaka Murata, Bull.Chem. Soc.Jap. 57 , 3597-3598 (1984)); Methylethylphosphinsäureanhydrid (vgl. z.B. DE-OS 31 01 427); Diphosphortetrajodid (Chem. Lett. 1983 , 449); Dialkyldisulfit (Indian J. Chem. 21 , 259 (1982)); oder andere reaktive Agentien.

Geeignete Lösungs-, Dispergier- oder Verdünnungsmittel für die Acylierung sind z.B. solche, die für die Durchführung der Cyclisierung angegeben worden sind, darüber hinaus auch z.B. Pyridin und Amide, wie z.B. Dimethylformamid. Neben Wasser werden für die Acylierung polare organische Lösungsmittel, wie Dimethylformamid, Dimethylsulfoxid oder Pyridin bevorzugt. Auch Lösungsmittelgemische, wie z.B. ein Gemisch aus Wasser und Methylenchlorid, sind geeignet.

Die erfindungsgemäßen Verbindungen der Formel I können gegebenenfalls in Form unterschiedlicher Stereoisomeren vorliegen. Die Erfindung umfaßt sowohl die möglichen einzelnen Stereoisomeren der Formel I als auch Mischungen mehrerer Stereoisomerer der Formel I mit beliebiger Zusammensetzung. Die Herstellung bestimmter Isomerer kann nach an sich bekannten Verfahren erfolgen. Beispielsweise können einzelne optisch aktive Isomere von Verbindungen der Formel I durch chirale Synthese oder Trennung von racemischen Gemischen erhalten werden.

Die substituierten 3-Amino-sydnonimine der allgemeinen Formel I können mit anorganischen oder organischen Säuren Säureadditionssalze bilden. Pharmakologisch annehmbare Säureadditionssalze werden bevorzugt. Zur Bildung derartiger Säureadditionssalze sind anorganische oder organische Säuren geeignet. Geeignete Säuren sind beispielsweise Chlorwasserstoff, Bromwasserstoff, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen-, Adipinsäure oder Naphthalindisulfonsäuren, insbesondere Naphthalindisulfonsäure (1,5). Die Säureadditionssalze können wie üblich durch Vereinigung der Komponenten, zweckmäßigerweise in einem geeigneten Lösungs- oder Verdünnungsmittel, hergestellt werden. Bei der Synthese der Verbindungen der Formel Ia fallen die Säureadditionssalze an. Gegebenenfalls können die freien Verbindungen der Formel I aus den Säureadditionssalzen in an sich bekannter Weise, z.B. durch Auflösen in Wasser und vorsichtige Zugabe einer Lauge, isoliert werden.

Die als Ausgangsprodukte benötigten substituierten N-Ni-trosoamino-acetonitrile der Formel II können dadurch hergestellt werden, daß

a) eine Verbindung der allgemeinen Formel IV

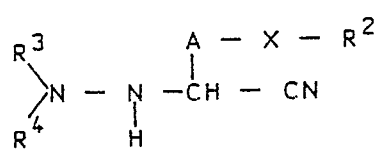

(IV)

worin A, X, $R^2$, $R^3$ und $R^4$ die bereits genannten Bedeutungen besitzen, nitrosiert wird, oder daß

b) ein Säureadditionssalz einer Verbindung der Formel Ia mit einer Base umgesetzt wird.

Die Verbindungen der Formel IV können in an sich bekannter Weise nach der Strecker'schen Aminonitrilsynthese aus Verbindungen der allgemeinen Formel V

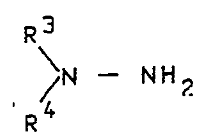

(V)

worin $R^3$ und $R^4$ die bereits genannten Bedeutungen besitzen, durch Umsetzung mit einem Aldehyd der allgemeinen Formel VI

$R^2$ - X - A - CHO (VI)

oder der maskierten Form eines solchen Aldehyds, z.B. einem Acetal der Formel VII

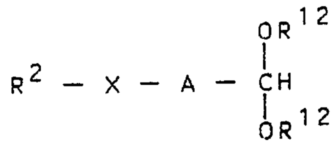

(VII)

worin $R^{12}$ einen geradkettigen oder verzweigten Alkylrest mit 1 bis 4 C-Atomen oder einen Benzylrest darstellt oder die beiden Reste $R^{12}$ zusammen eine gegebenenfalls durch Alkylgruppen substituierte Di- oder Trimethylenbrücke darstellen, und Blausäure bzw. einem geeigneten Cyanid, z.B. Natriumcyanid oder einem Silylcyanid, in einem geeigneten Lösungsmittel, z.B. Wasser, hergestellt werden.

Die Nitrosierung der Verbindung der Formel IV wird in bekannter Weise, zweckmäßigerweise in einem geeigneten inerten Lösungsmittel oder Lösungsmittelgemisch, vorzugsweise in Wasser, normalerweise bei Temperaturen von 0 bis 40°C und vorzugsweise bei Temperaturen von 0 bis 10°C, durchgeführt. Die Nitrosierung erfolgt z.B. mit salpetriger Säure, NO, NOCl oder NO-haltigen Gasgemischen. Zweckmäßigerweise erfolgt die Nitrosierung mit salpetriger Säure, die vorteilhafterweise aus einem Alkalimetallnitrit, z.B. Natriumnitrit, und einer Säure, insbesondere Salzsäure, erzeugt wird. Es ist zweckmäßig, die wäßrige Lösung der Verbindung IV mit einer Säure, insbesondere Salzsäure, auf einen pH-Wert von 1 bis 3 einzustellen und das Alkalimetallnitrit in Form einer wäßrigen Lösung zu der gerührten und abgekühlten Lösung der Verbindung zuzutropfen.

Aus der Lösung der dabei erhaltenen Verbindung II kann die Verbindung II isoliert werden, oder die Lösung der Verbindung II kann direkt der Cyclisierungsreaktion unterworfen werden. Normalerweise ist es jedoch für die anschließende Cyclisierung angebracht, die Nitrosoverbindung II zunächst in einem geeigneten organischen Lösungsmittel aufzunehmen und in ihm, gegebenenfalls nach Zusatz eines weiteren Lösungsmittels, die Cyclisierung zu der Verbindung der Formel Ia durchzuführen.

Die Verbindungen der allgemeinen Formel V sind zum Teil bekannt bzw. lassen sich, ausgehend von Verbindungen der allgemeinen Formel VIII

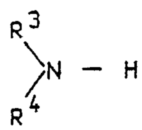

(VIII)

dadurch herstellen, daß entweder

a) eine Verbindung der Formel VIII zur N-Nitrosoverbindung VIIIa nitrosiert und diese dann mit einem geeigneten Reduktionsmittel, beispielsweise Lithiumaluminiumhydrid, reduziert wird:

(VIII) 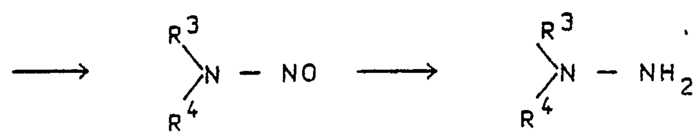

(VIIIa) (V)

oder daß in an sich bekannter Weise

b) eine Verbindung der Formel VIII mit Kaliumcyanat im sauren Medium in das Harnstoffderivat überführt und dieses dann durch Oxidation mit Natriumhypochlorit nach dem Hoffmann-Abbau in die Verbindung V überführt wird:

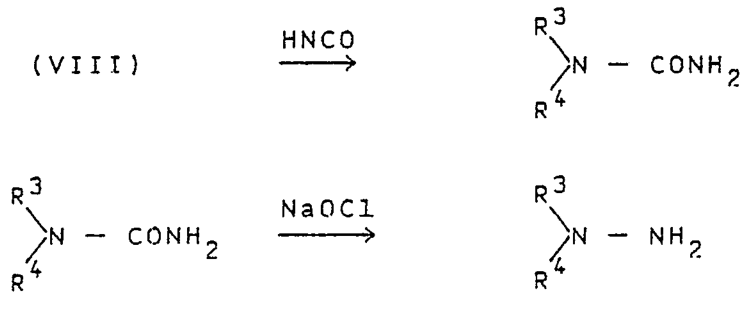


(VIII)

(V)

Verbindungen der allgemeinen Formel VI sind zum Teil bekannt oder lassen sich in an sich bekannter Weise beispielsweise dadurch herstellen, daß entweder

a) ein Acetal der Formel VII im sauren Medium gespalten wird

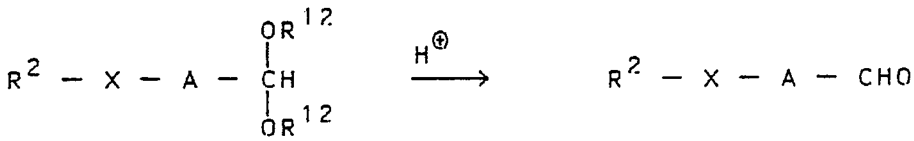

(VII) (VI)

oder daß

b) ein Nucleophil der Formel IX, worin $R^2$ und X die bereits genannte Bedeutung haben, an die Doppelbindung eines Aldehyds der Formel X addiert wird, wobei A' einen Alkenylrest darstellt und durch formales Hinzufügen eines Wasserstoffatoms in die Alkylenkette A mit ihrer bereits genannten Bedeutung übergeht:

$$R^2 - X - H \quad + \quad A' - CHO \longrightarrow R^2 - X - A - CHO$$

(IX) (X) (VI)

Verbindungen der allgemeinen Formel VII sind zum Teil bekannt oder lassen sich in an sich bekannter Weise beispielsweise dadurch herstellen, daß entweder

a) ein Acetal der Formel XI, worin Z eine nucleofuge Gruppe darstellt, beispielsweise ein Chlor- oder ein Bromatom, mit einem Nucleophil der Formel IX umgesetzt wird:

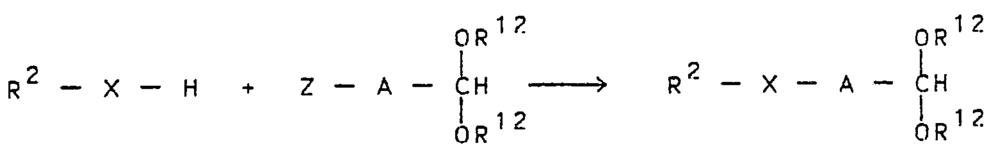

(IX) (XI) (VII)

oder daß

b) ein Acetal der Formel XIII mit einem Elektrophil der Formel XII, worin Z die bereits genannte Bedeutung hat, alkyliert wird:

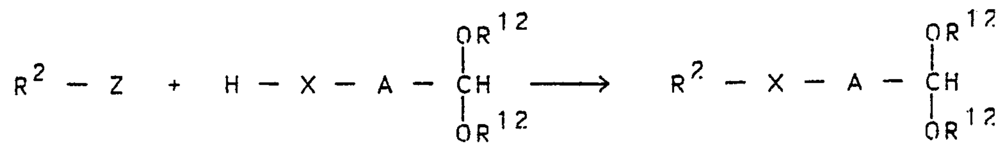

(XII) (XIII) (VII)

Verbindungen der Formel II können auch dadurch hergestellt werden, daß ein Säureadditionssalz einer Verbindung der Formel Ia, zweckmäßigerweise in wäßriger Lösung, mit einer Base, d.h. einer Verbindung, die in Wasser eine alkalische Reaktion einstellt, wie z.B. einem Alkalihydroxid, wie z.B. Lithium-, Natrium- oder Kaliumhydroxid, einem Alkalikarbonat, wie z.B. Lithium-, Kalium- oder Natriumkarbonat oder einem Alkalibikarbonat, wie z.B. Natriumbikarbonat, oder einem Amin, insbesondere einem tertiären Amin, wie z.B. Triethylamin, behandelt wird. Die Umsetzung wird normalerweise bei 10 bis 40°C, vorzugsweise bei Raumtemperatur, durchgeführt. Es wird mindestens so viel Base zugesetzt, daß der Säurerest vollständig gebunden wird. In der Regel wird das Säureadditionssalz in Wasser oder einem Gemisch aus Wasser und einem Lösungsmittel gelöst und so viel Base zugesetzt, bis die wäßrige Lösung alkalisch reagiert. Die Bindung des Säurerestes kann auch mit einem Austauscherharz erfolgen.

Auch die Verbindungen der Formel II können mit anorganischen oder organischen Säuren Säureadditionssalze bilden, von denen pharmakologisch annehmbare Säureadditionssalze bevorzugt sind. Bezüglich der Bildung dieser Säureadditionssalze und geeigneter Säuren gilt das bereits bei den Säureadditionssalzen der Verbindung I Gesagte.

Die Verbindungen der allgemeinen Formeln I und II und ihre pharmakologisch annehmbaren Säureadditionssalze besitzen wertvolle pharmakologische Eigenschaften. Besonders ausgeprägt ist ihre Wirkung auf das Herz-Kreislauf-System.

Verglichen mit bekannten, in der 4-Position unsubstituierten 3-Aminosydnoniminen, z.B. dem Handelsprodukt Molsidomin, oder in der 4-Position durch eine Alkyl- oder Aralkylgruppe substituierten 3-Aminosydnoniminen weisen die im 4-Substituenten eine Ether- oder Thioethergruppe enthaltenden Verbindungen der allgemeinen Formel I eine längere Wirkdauer und/oder höhere Wirkstärke auf. Gleiches gilt für die Verbindungen der Formel II im Vergleich mit anderen N-Nitroso-aminoacetonitrilen. Die Verbindungen der Formeln I und II und ihre pharmakologisch annehmbaren. Säureadditionssalze senken beispielsweise den Blutdruck ebenso wie den Pulmonalarteriendruck und den linksventrikulären enddiastolischen Druck und tragen so zu einer Entlastung der Herztätigkeit im Sinne einer antianginösen Wirkung bei, ohne dabei eine reflektorische Tachykardie zu provozieren.

Die Verbindungen der Formeln I und II und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formeln I und/oder II oder eines Säureadditionssalzes davon neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen

der Formeln I und/oder II oder ihrer pharmakologisch annehmbaren Säureadditionssalze und noch andere therapeutisch wirksame Stoffe enthalten.

Derartige andere therapeutisch wirksame Substanzen sind beispielsweise: β-Rezeptorenblocker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

In den pharmazeutischen Präparaten kann der Gehalt an dem Wirkstoff oder den Wirkstoffen der Formel I in weiten Grenzen schwanken und z.B. 0,05 bis 50 Gew.%, vorzugsweise 0,05 bis 20 Gew.% betragen. In festen Darreichungsformen, wie Dragees, Tabletten etc. beträgt der Gehalt an einem oder mehreren Wirkstoffen der Formel I in vielen Fällen 2 bis 20 Gew.%. Flüssige Darreichungsformen, wie Tropfen, Emulsionen und Injektionslösungen enthalten häufig 0,05 bis 2 Gew.%, vorzugsweise 0,05 bis. 1 Gew.% eines oder mehrerer Wirkstoffe der Formel I. Der Gehalt an einem oder mehreren Wirkstoffen der Formel I kann in den pharmazeutischen Präparaten teilweise, z.B. bis zu 50 Gew.%, vorzugsweise zu 5 bis 40 Gew.% durch eine oder mehrere andere therapeutisch wirksame Substanzen ersetzt sein.

Die Verbindungen der Formeln I und/oder II, ihre pharmakologisch annehmbaren Säureadditionssalze und pharmazeutische Präparate, welche die Verbindungen der Formeln I und/oder II oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 500 mg, vorzugsweise 1 bis 100 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Die pharmakologische Wirkung der Verbindungen der Formel I wurde nach einer modifizierten Methode von Godfraind and Kaba (Arch. Int. Pharmacodyn. Ther. 196 , (Suppl) 35 bis 49, 1972) und von Schüman et al (Naunyn-Schmiedeberg's Arch. Pharmacol. 289 , 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l $CaCl_2$ löst dann eine Kontraktion aus. Die relaxierende Wirkung der Prüfsubstanz wird durch kumulative Zugabe in halblogarithmisch abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50 % hemmt (= $IC_{50}$, mol/l). In der folgenden Tabelle sind die so erhaltenen $IC_{50}$-Werte angegeben. Wie der Vergleich mit dem $IC_{50}$-Wert $>3 \cdot 10^{-4}$ für die bekannte Verbindung Molsidomin (N-Ethoxy-carbonyl-3-morpholinosydnonimin), vgl. DE-B-16 95 897,. ergibt, liegen die Werte für die Verbindungen der Formel I erheblich günstiger.

TABELLE

| Verbindung (Beispiel-Nr.) | $IC_{50}$ (mol/l) |
|---|---|
| 5 | $1.1 \cdot 10^{-6}$ |
| 7 | $0.7 \cdot 10^{-6}$ |
| 8 | $0.8 \cdot 10^{-6}$ |
| 10 | $2.0 \cdot 10^{-6}$ |
| 12 | $0.5 \cdot 10^{-6}$ |
| 13 | $2.0 \cdot 10^{-6}$ |
| Molsidomin | $>3.0 \cdot 10^{-4}$ |

Beispiel 1

3-(2, 6-Dimethylpiperidino)-4-ethylthiomethyl-sydnoniminhydrochlorid

a) 2-(2,6-Dimethylpiperidino)amino-3-ethylthiopropionitril

Zur Lösung von 16,5 g 1-Amino-2,6-dimethylpiperidinhydrochlorid in 50 ml Wasser wird bei 0 bis 5°C eine Lösung von 4,9 g Natriumcyanid in 20 ml Wasser getropft, dann werden in 30 min 11,4 g Ethylthioacetaldehyd zugetropft. Der pH-Wert des Reaktionsgemisches wird mit Salzsäure auf 7 eingestellt. Nach Rühren über Nacht wird mit Dichlormethan extrahiert, die vereinigten Extrakte werden getrocknet und im Rotationsverdampfer eingeengt. Es werden 21,6 g 2-(2,6-Dimethylpiperidino)amino-3-ethylthiopropionitril erhalten, die ohne weitere Reinigung in die Folgestufe eingesetzt werden.

b) 3-(2,6-Dimethylpiperidino)-4-ethylthiomethyl-sydnoniminhydrochlorid

Zur Mischung von 21,6 g des unter a) beschriebenen Ethylthiopropionitrils, 50 ml Wasser und 5,1 ml konzentrierter Salzsäure wird unter Eiskühlung eine Lösung von 3,9 g Natriumnitrit in 20 ml Wasser getropft. Nach 2-stündigem Rühren bei 0-5°C wird mit Ethylacetat extrahiert, die vereinigten Extrakte werden getrocknet und teilweise eingengt. Nach Zugabe von 100 ml methanolischer Chlorwasserstofflösung wird 2 Stunden unter Eiskühlung Chlorwassserstoff eingeleitet. Die Lösung wird im Rotationsverdampfer eingeengt, der Rückstand mit Aceton verrührt, der Niederschlag abgesaugt, das Filtrat im Rotationsverdampfer eingeengt, der Rückstand mehrmals mit Ethylacetat verrührt und der Niederschlag abgesaugt. Die vereinigten Niederschläge werden aus Acetonitril umkristallisiert. Es werden 7,7 g 3-(2,6-Dimethylpiperidino)-4-ethylthiomethylsydnoninimhydrochlorid vom Schmelzpunkt 132°C (Zers.) erhalten.

Beispiel 2

3-(2,6-Dimethylpiperidino)-N-ethoxycarbonyl-4-ethylthiomethylsydnonimin

Zu 2,5 g des unter 1) beschriebenen 3-(2,6-Dimethylpiperidino)-4-ethylthiomethylsydnoniminhydrochlorids in 25 ml Wasser und 10 ml Dichlormethan werden unter Eiskühlung 1,5 g Natriumhydrogencarbonat gegeben, dann wird eine Lösung von 1,0 g Chlorameisensäureethylester in 5 ml Dichlormethan zugetropft. Nach 2-stündigem Rühren wird die organische Phase abgetrennt, die wäßrige Phase wird mit Dichlormethan extrahiert, die vereinigten organischen Phasen werden getrocknet und im Rotationsverdampfer eingeengt. Der Rückstand wird aus Hexan umkristallisiert. Ausbeute: 2,3 g 3-(2,6-Dimethylpiperidino)-N-ethoxycarbonyl-4-ethylthiomethylsydnonimin vom Schmelzpunkt 54°C.

Beispiel 3

N-Benzoyl-3-(2,6-dimethylpiperidino)-4-ethylthiomethylsydnonimin

Analog 2) werden 2,5 g des unter 1) beschriebenen 3-(2,6-Dimethylpiperidino)-4-ethylthiomethylsydnoniminhydrochlorids mit 1,3 g Benzoylchlorid in Dichlormethan/Wasser in Gegenwart von 1,4 g Natriumhydrogencarbonat acyliert. Das erhaltene Produkt wird durch Chromatographie an Kieselgel mit Dichlormethan/Methanol (98:2) gereinigt. Es werden 0,7 g N-Benzoyl-3-(2,6-dimethylpiperidino)-4-ethylthiomethylsydnonimin als farbloses Öl erhalten.

Beispiel 4

4-Allylthiomethyl-3-(2,6-dimethylpiperidino)sydnoniminhydrochlorid

a) 3-Allylthio-2-((2,6-dimethylpiperidino)amino)-propionitril

Zur Mischung aus 6,0 g 1-Amino-2,6-dimethylpiperidin, 30 ml Wasser und 5,6 ml konzentrierter Salzsäure wird eine Lösung von 9,5 g Allylthioacetaldehydiethylacetal in 10 ml Methanol getropft. Nach 2-stündigem Rühren wird bei 0°C eine Lösung von 2,5 g Natriumcyanid in 20 ml Wasser zugetropft. Der pH-Wert wird mit Natriumhydrogencarbonatlösung auf 7 eingestellt und das Reaktionsgemisch über Nacht gerührt. Es wird mit Dichlormethan extrahiert, die vereinigten Extrakte werden getrocknet und im Rotationsverdampfer eingeengt. Es werden 12,3 g 3-Allylthio-2-((2,6-dimethylpiperidino)amino)propionitril erhalten, die ohne weitere Reinigung in die Folgestufe eingesetzt werden.

b) 4-Allylthiomethyl-3-(2,6-dimethylpiperidino)sydnoniminhydrochlorid

Zur Mischung von 12,0 g der unter a) beschriebenen Aminonitrilzwischenstufe, 50 ml Wasser und 4,1 ml konzentrierter Salzsäure wird bei 0-5°C eine Lösung von 3,3 g Natriumnitrit in 20 ml Wasser getropft. Nach 3-stündigem Rühren bei 0°C wird mit Ethylacetat extrahiert, die vereinigten Extrakte werden getrocknet, teilweise eingeengt und mit 50 ml methanolischer Chlorwasserstofflösung versetzt. Es wird 1,5 h Chlorwasserstoff eingeleitet, die Lösung im Rotationsverdampfer eingeengt, der Rückstand mit Acetonitril verrührt, der Niederschlag abgesaugt, das Filtrat im Rotationsverdampfer eingeengt und der Rückstand an Kieselgel mit Dichlormethan/Methanol (9:1) chromatographiert. Nach Verreiben mit Aceton/Diethylether und Absaugen werden 2,2 g 4-Allylthiomethyl-3-(2,6-dimethylpiperidino)sydnoniminhydrochlorid erhalten, die bei 151-152°C (Zers.) schmelzen.

Beispiel 5

3-(2,6-Dimethylpiperidino)-4-phenylthiomethylsydnoniminhydrochlorid

a) 2-(2,6-Dimethylpiperidino)amino-3-phenylthiopropionitril

Analog 1a) werden 8,6 g 1-Amino-2,6-dimethylpiperidinhydrochlorid, 2,6 g Natriumcyanid und 7,9 g'Phenylthioacetaldehyd umgesetzt. Es werden 13,0 g 2-(2,6-Dimethylpiperidino)amino-3-phenylthiopropionitril erhalten, die ohne weitere Reinigung in die Folgestufe eingesetzt werden.

b) 3-(2,6-Dimethylpiperidino)-4-phenylthiomethylsydnoniminhydrochlorid

Analog 1b) werden 13,0 g der unter a) beschriebenen Propionitrilzwischenstufe in Gegenwart von 3,9 ml konzentrierter Salzsäure mit 3,1 g Natriumnitrit umgesetzt. Das nach Cyclisierung mit Chlorwasserstoff erhaltene Rohprodukt wird an Kieselgel mit Dichlormethan/Methanol (9:1) chromatographiert und aus Aceton/Acetonitril umkristallisiert.
Ausbeute: 4,3 g 3-(2,6-Dimethylpiperidino)-4-phenylthiomethylsydnoniminhydrochlorid vom Schmelzpunkt 164-165°C (Zers.)

Beispiel 6

N-Benzoyl-3-(2,6-dimethylpiperidino)-4-phenylthiomethylsydnonimin

Analog 3) werden aus 1,4 g des unter 5) beschriebenen 3-(2,6-Dimethylpiperidino)-4-phenylthiomethylsydnoniminhydrochlorids, 0,6 g Benzoylchlorid und 0,7 g Natriumhydrogencarbonat in Dichlormethan/Wasser 0,6 g N-Benzoyl-3-(2;6-dimethylpiperidino)-4-phenylthiomethylsydnonimin als Öl erhalten.

Beispiel 7

4-Benzylthiomethyl-3-morpholinosydnoniminhydrochlorid

a) 3-Benzylthio-2-(morpholinoamino)propionitril

Zur Mischung aus 10,3 g 4-Aminomorpholin, 50 ml Wasser und 8,6 ml konzentrierter Salzsäure wird unter Eiskühlung eine Lösung von 4,9 g Natriumcyanid in 30 ml Wasser getropft. Dann wird eine Lösung von 16,6 g Benzylthioacetaldehyd in 20 ml Methanol zugetropft und das Reaktionsgemisch über Nacht gerührt. Das Methanol wird am Rotationsverdampfer abgezogen, die wäßrige Lösung wird mit Dichlormethan extrahiert, die vereinigten Extrakte werden getrocknet und im Rotationsverdampfer eingeengt. Es verbleiben 24,9 g 3-Benzylthio-2-(morpholinoamino)propionitril, die ohne weitere Reinigung in die Folgestufe eingesetzt werden.

b) 4-Benzylthiomethyl-3-morpholinosydnoniminhydrochlorid

Zur Mischung von 24,8 g des unter a) beschriebenen Benzylthiopropionitrils, 50 ml Wasser und 7,7 ml konzentrierter Salzsäure wird bei 0-5°C eine Lösung von 6,2 g Natriumnitrit in 20 ml Wasser getropft. Nach 1-stündigem Rühren wird der pH-Wert mit Natriumhydrogencarbonatlösung auf 5 gestellt, die Lösung wird mit Ethylacetat extrahiert, die vereinigten Extrakte werden getrocknet, teilweise eingeengt und mit 100 ml methanolischer Chlorwasserstofflösung versetzt. Es wird 5 h unter Eiskühlung Chlorwasserstoff eingeleitet, teilweise eingeengt, der ausgefallene Niederschlag abgesaugt, das Filtrat weiter eingeengt, der Niederschlag abgesaugt und mit Aceton verrührt. Der Rückstand wird aus Isopropanol umkristallisiert.
Ausbeute: 6,9 g 4-Benzylthiomethyl-3-morpholinosydnoniminhydrochlorid als Hemihydrat vom Schmelzpukt 165-167°C (Zers.).

Beispiel 8

4-Benzylthiomethyl-3-(2,6-dimethylpiperidino)sydnoniminhydrochlorid

a) 3-Benzylthio-2-((2,6-dimethylpiperidino)amino)propionitril

Zur Mischung aus 7,4 g 1-Amino-2,6-dimethylpiperidin, 60 ml Wasser und 4,9 ml konzentrierter Salzsäure werden eine Lösung von 2,8 g Natriumcyanid in 20 ml Wasser und eine Lösung von 9,6 g Benzylthioacetaldehyd in 20 ml Methanol getropft. Der pH-Wert wird mit Salzsäure auf 7 gebracht, das Reaktionsgemisch über Nacht gerührt und mit Dichlormethan extrahiert. Die vereinigten Extrakte werden mit essigsaurem Wasser vom pH 4-5 gewaschen, getrocknet und im Rotationsverdampfer eingeengt. Es werden 14,9 g 3-Benzylthio-2-((2,6-dimethylpiperidino)amino)propionitril erhalten, die ohne weitere Reinigung weiter umgesetzt werden.

b) 4-Benzylthiomethyl-3-(2,6-dimethylpiperidino)sydnoniminhydrochlorid

Zur Mischung von 14,8 g des unter a) beschriebenen Aminopropionitrils, 50 ml Wasser und 4,3 ml konzentrierter Salzsäure wird unter Eiskühlung eine Lösung von 3,6 g Natriumnitrit in 25 ml Wasser getropft. Nach 2-stündigem Rühren bei 0-5°C wird mit Ethylacetat extrahiert, die vereinigten Extrakte werden getrocknet, teilweise eingeengt und mit 50 ml methanolischer Chlorwasserstofflösung versetzt. Es wird 1 h unter Eiskühlung Chlorwasserstoff eingeleitet. Der ausgefallene Niederschlag wird abgesaugt, das Filtrat im Rotationsverdampfer eingeengt und der Rückstand an Kieselgel mit Dichlormethan/Methanol (95:5) chromatographiert. Es werden 6,2 g 4-Benzylthiomethyl-3-(2,6-dimethylpiperidino)-sydnoniminhydrochlorid erhalten, die nach Umkristallisation aus Aceton bei 150 bis 152°C unter Zersetzung schmelzen.

Beispiel 9

4-Benzylthiomethyl-3-(2,6-dimethylpiperidino)-N-ethoxycarbonylsydnonimin

Analog 3) werden 2,7 g des unter 8) beschriebenen 4-Benzylthiomethyl-3-(2,6-dimethylpiperidino)-sydnoniminhydrochlorids in Gegenwart von 1,2 g Natriumhydrogencarbonat mit 1,0 g Chlorameisensäureethylester umgesetzt. Es werden 2,4 g 4-Benzylthiomethyl-3-(2,6-dimethylpiperidino)-N-ethoxycarbonylsydnonimin als Öl erhalten.

Beispiel 10

N-Benzoyl-4-benzylthiomethyl-3-(2,6-dimethylpiperidino)sydnonimin

Analog 2) werden 2,0 g des unter 8) beschriebenen 4-Benzylthiomethyl-3-(2,6-dimethylpiperidino) sydnoniminhydrochlorids in Gegenwart von 1,0 g Natriumhydrogencarbonat mit 0,9 g Benzoylchlorid umgesetzt. Durch Umkristallisation aus Hexan werden 1,7 g N-Benzoyl-4-benzylthiomethyl-3-(2,6-dimethylpiperidino)sydnonimin vom Schmelzpunkt 98 bis 100°C erhalten.

Beispiel 11

4-Benzylthiomethyl-3-(2,6-dimethylpiperidino)-N-(4-methoxybenzoyl sydnonimin

Analog 3) werden 2,1 g des unter 8) beschriebenen 4-Benzylthiomethyl-3-(2,6-dimethylpiperidino)-sydnoniminhydrochlorids in Gegenwart von 1,1 g Natriumhydrogencarbonat mit 1,4 g 4-Methoxybenzoylchlorid umgesetzt. Es werden 0,7 g 4-Benzylthiomethyl-3-(2,6-dimethylpiperidino)-N-(4-methoxybenzoyl)-sydnonimin vom Schmelzpunkt 104°C erhalten.

Beispiel 12

4-(2-Benzylthio-1-methylethyl)-3-morpholinosydnoniminhydrochlorid

a) 4-Benzylthio-2-morpholino-amino-3-methyl-butyronitril

Analog 7a) werden 5,8 g 4-Aminomorpholin, 2,7 g Natriumcyanid und 10,7 g 3-Benzylthio-2-methylpropionaldehyd umgesetzt. Es werden 16,1 g 4-Benzylthio-2-morpholinoamino-3-methylbutyronitril erhalten, die ohne weitere Reinigung in die Folgestufe eingesetzt werden.

b) 4-(2-Benzylthio-1-methylethyl)-3-morpholinosydnoniminhydrochlorid

Analog 7b) werden 16,0 g des unter a) beschriebenen Benzylthiobutyronitrils in Gegenwart von 4,3 ml konzentrierter Salzsäure mit 3,6 g Natriumnitrit umgesetzt. Das nach Cyclisierung mit Chlorwasserstoff erhaltene Rohprodukt wird an Kieselgel mit Dichlormethan/Methanol (7:1) chromatographiert. Es werden 2,0 g 4 4-(2-Benzylthio-1-methylethyl)-3-morpholinosydnoniminhydrochlorid erhalten, die nach Umkristallisation aus Isopropanol/Diethylether bei 173°C unter Zersetzung schmelzen.

Beispiel 13

3-(2,6-Dimethylpiperidino)-4-phenoxymethylsydnoniminhydrochlorid

a) 2-(2,6-Dimethylpiperidino)amino-3-phenoxypropionitril

Analog 8a) werden 12,8 g 1-Amino-2,6-dimethylpiperidin, 5,4 g Natriumcyanid und 15,0 g Phenoxyacetaldehyd umgesetzt. Es werden 20,8 g 2-(2,6-Dimethylpiperidino)amino-3-phenoxypropionitril erhalten, die ohne weitere Reinigung in die Folgestufe eingesetzt werden.

b) 3-(2,6-Dimethylpiperidino)-4-phenoxymethylsydnoniminhydrochlorid

Analog 8b) werden 20,7 g des unter a) beschriebenen Phenoxypropionitrils in Gegenwart von 6,5 ml konzentrierter Salzsäure mit 5,2 g Natriumnitrit umgesetzt. Das nach Cyclisierung mit Chlorwasserstoff erhaltene Rohprodukt wird an Kieselgel mit Dichlormethan/Methanol (9:1) chromatographiert. Es werden 4,7 g 3-(2,6-Dimethylpiperidino)amino-4-phenoxymethylsydnoniminhydrochlorid erhalten, die nach Umkristallisation aus Isopropanol bei 160-161°C unter Zersetzung schmelzen.

Beispiel 14

4-Benzyloxymethyl-3-(2,6-dimethylpiperidino)sydnoniminhydrochlorid

a) 3-Benzyloxy-2-((2,6-dimethylpiperidino)amino)propionitril

Analog 8a) werden 12.8 g 1-Amino-2,6-dimethylpiperidin, 4,9 g Natriumcyanid und 15,0 g Benzyloxyacetaldehyd umgesetzt. Es werden 23,3 g 3-Benzyloxy-2-((2,6-dimethylpiperidino)amino)propionitril erhalten, die ohne weitere Reinigung in die Folgestufe eingesetzt werden.

b) 4-Benzyloxymethyl-3-(2,6-dimethylpiperidino)sydnoniminhydrochlorid

Analog 8b) werden 23,0 g des unter a) beschriebenen Benzyloxypropionitrils in Gegenwart von 6,9 ml konzentrierter Salzsäure mit 5,5 g Natriumnitrit umgesetzt. Das nach Cyclisierung mit Chlorwasserstoff erhaltene Rohprodukt wird an Kieselgel mit Dichlormethan/Methanol (9:1) chromatographiert. Es werden 4,0 g 4-Benzyloxymethyl-3-(2,6-dimethylpiperidino)sydnoniminhydrochlorid als Öl erhalten.

Beispiel 15

4-Benzyloxymethyl-3-(2,6-dimethylpiperidino)-N-ethoxy carbonylsydnonimin

Analog 3) werden 3,6 g des unter 14) beschriebenen 4-Benzyloxymethyl-3-(2,6-dimethylpiperidino) sydnoniminhydrochlorids in Gegenwart von 1,8 g Natriumhydrogencarbonat mit 1,3 g Chlorameisensäureethylester umgesetzt. Es werden 2,8 g 4-Benzyloxymethyl-3-(2,6-dimethylpiperi dino)-N-ethoxycarbonylsydnonimin erhalten, die nach Verrühren mit Hexan bei 56 bis 57°C schmelzen.

In den nachfolgenden Beispielen A bis F werden pharmazeutische Präparate beschrieben.

| Beispiel A | |
|---|---|
| Gelatineweichkapseln, enthaltend 5 mg Wirkstoff pro Kapsel: | |
| | pro Kapsel |
| Wirkstoff | 5 mg |
| Aus Kokosfett fraktioniertes Triglycerid-Gemisch | 150 mg |
| Kapselinhalt | 155 mg |

| Beispiel B | |
|---|---|
| Injektionslösung, enthaltend 1 mg Wirkstoff pro ml: | |
| | pro ml |
| Wirkstoff | 1,0 mg |
| Polyethylenglycol 400 | 0,3 ml |
| Natriumchlorid | 2,7 mg |
| Wasser zu Injektionszwecken | ad 1 ml |

| Beispiel C | |
|---|---|
| Emulsion, enthaltend 3 mg Wirkstoff pro 5 ml | |
| | pro 100 ml Emulsion |
| Wirkstoff | 0,06 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

| Beispiel D | |
|---|---|
| Rektale Arzneiform, enthaltend 4 mg Wirkstoff pro Suppositorium | |
| | pro Suppositorium |
| Wirkstoff | 4 mg |
| Suppositoriengrundmasse | ad 2 g |

| Beispiel E | |
|---|---|
| Tabletten, enthaltend 2 mg Wirkstoff pro Tablette | |
| | pro Tablette |
| Wirkstoff | 2 mg |
| Lactat (feingemahlen) | 2 mg |
| Maisstärke (weiß) | 150 mg |
| Milchzucker | 60 mg |
| Mikrokristalline Cellulose | 50 mg |
| Polyvinylpyrrolidon | 20 mg |
| Magnesiumstearat | 2 mg |
| Natriumcarboxymethylstärke | 25 mg |
| | 311 mg |

| Beispiel F | |
|---|---|
| Dragees, enthaltend 1 mg Wirkstoff pro Dragee | |
| | pro Dragee |
| Wirkstoff | 1 mg |
| Maisstärke | 100 mg |
| Lactose | 60 mg |
| sec Calciumphosphat | 30 mg |
| lösliche Stärke | 3 mg |
| Magnesiumstearat | 2 mg |
| kolloidale Kieselsäure | 4 mg |
| | 200 mg |

## Ansprüche

1. Substituierte 3-Aminosydnonimine der allgemeinen Formel I

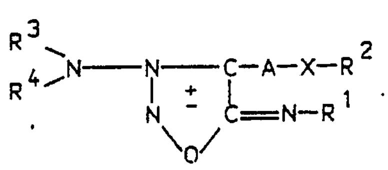

(I)

und ihre pharmakologisch annehmbaren Säureadditionssalze,
worin
A eine geradkettige oder verzweigte Alkylenkette mit 1 bis 6 C-Atomen;
X eine der Gruppen -O- oder -S- ;
$R^1$ Wasserstoff oder den Rest $-COR^5$;
$R^2$ eine Alkylgruppe mit 1 bis 6 C-Atomen, eine Alkenyl-oder Alkinylgruppe mit jeweils 3 bis 6 C-Atomen, eine gegebenenfalls substituierte Aralkylgruppe mit 1 bis 4 C-Atomen in der Alkyl- und 6 bis 10 C-Atomen in der Arylgruppe oder eine gegebenenfalls substituierte Arylgruppe mit 6 bis 10 C-Atomen;

$R^3$ und $R^4$, die gleich oder verschieden sein können, Alkylgruppen mit 1 bis 6 C-Atomen, Alkenyl-, Alkinyl- oder Cycloalkylgruppen mit jeweils 3 bis 6 C-Atomen, gegebenenfalls substituierte Aralkylgruppen mit 1 bis 4 C-Atomen in der Alkylgruppe und 6 bis 10 C-Atomen in der Arylgruppe, gegebenenfalls substituierte Arylgruppen mit 6 bis 10 C-Atomen, heterocyclische Gruppen mit 3 bis 5 Ring-C-Atomen und der Gruppe $-S(O)_m-$ oder $R^3$ und $R^4$ zusammen mit dem Stickstoffatom, an das sie gebunden sind, einen heterocyclischen Ring der Formel

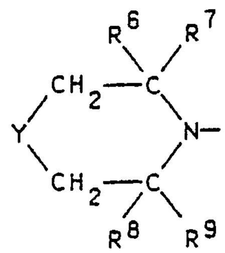

;

$R^5$ einen aliphatischen Rest mit 1 bis 6 C-Atomen, der auch durch Alkoxy mit 1 bis 6 C-Atomen oder durch einen aliphatischen Thiorest mit bis zu 4 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen, der auch durch Alkoxy mit 1 bis 6 C-Atomen substituiert sein kann; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 4 C-Atomen und/oder 1 bis 3 Alkoxyreste mit 1 bis 3 C-Atomen und/oder 1 oder 2 Nitrogruppen und/oder 1 oder 2 Hydroxygruppen und/oder 1 oder 2 Alkylcarbonyloxyreste mit 1 bis 4 C-Atomen und/oder 1 bis 3 Alkylthioreste mit 1 bis 4-C-Atomen und/oder einen Trifluormethylrest und/oder einen Imidazolylrest mono-, di- oder trisubstituierten Arylrest mit 6 bis 10 C-Atomen; Imidazolyl; Pyridyl; Thienyl; Styryl;

m eine der Zahlen 0, 1 oder 2;

Y eine der Gruppen $-(CH_2)_n-$, -O-, $-S(O)_n-$ oder $-N(R^{10})-$;

$R^6$, $R^7$, $R^8$ und $R^9$, die gleich oder verschieden sein können, Wasserstoff oder Alkylgruppen mit 1 bis 4 C-Atomen;

n eine der Zahlen 0, 1 oder 2;

$R^{10}$ einen Alkylrest mit 1 bis 4 C-Atomen, einen gegebenenfalls substituierten Arylrest mit 6 bis 12 C-Atomen oder einen Rest der Formel $-COOR^{11}$, -COH, $-COR^{11}$ oder $-S(O)_2R^{11}$;

$R^{11}$ einen Alkylrest mit 1 bis 4 C-Atomen

bedeuten.

2. Substituierte 3-Aminosydnonimine nach Anspruch 1, dadurch gekennzeichnet, daß $R^3$ und $R^4$ zusammen mit dem stickstoffatom, an das sie gebunden sind, einen hetero cyclischen Ring der Formel

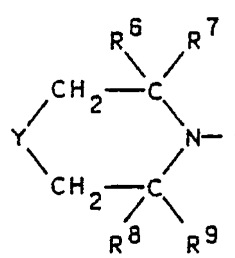

bedeuten, wobei Y vorzugsweise eine der Gruppen $-(CH_2)_o-$, $-(CH_2)_j$, -O-, -S- oder $-S(O)_2$-bedeutet, und/oder $R^6$, $R^7$, $R^8$ und $R^9$, die gleich oder verschieden sein können, Wasserstoffatome oder Methylgruppen bedeuten, und/oder $R^2$ einen Alkylest mit 1 bis 6 C-Atomen, einen Alkenyl- oder Alkinylest mit jeweils 3 bis 5 C-Atomen, einen Aralkylrest mit 1 bis 3 C-Atomen in der Alkylgruppe und einem unsubstituierten, mono- oder disubstituierten Phenylrest als Arylgruppe oder einen unsubstituierten, mono- oder disubstituierten Phenylrest bedeutet.

3. Substituierte 3-Aminosydnonimine nach Anspruch 1 und/oder 2, dadurch gekennzeichnet, daß $R^2$ einen Alkylrest mit 1 bis 4 C-Atomen, einen Alkenylrest mit 3 bis 5 C-Atomen, einen Phenalkylrest mit 1 bis 3 C-Atomen in der Alkylgruppe oder einen Phenylrest bedeutet.

4. Substituierte 3-Aminosydnonimine nach einem oder mehreren der Ansprüche 1 bis 3, dadurch gekennzeichnet, daß
$R^2$ Ethyl, Phenyl, Benzyl oder Allyl,
$R^3$ und $R^4$ zusammen mit dem Stickstoffatom, an das sie gebunden sind, Morpholino oder 2,6-Dimethylpiperidino,
$R^5$ Ethoxy, Phenyl oder Methoxyphenyl und
A Methylen oder 1-Methyl-ethylen
bedeuten.
5. 4-Benzylthiomethyl-3-(2,6-dimethylpiperidino)sydnonimin und seine pharmakologisch annehmbaren Säureadditionssalze, insbesondere sein Hydrochlorid.
6. 3-(2,6-Dimethylpiperidino)-4-phenoxymethylsydnonimin und seine pharmakologisch annehmbaren Säureadditionssalze, insbesondere sein Hydrochlorid.
7. Verfahren zur Herstellung der in einem oder mehreren der Ansprüche 1 bis 6 angegebenen Verbindungen der Formel I, dadurch gekennzeichnet, daß eine Verbindung der allgemeinen Formel II

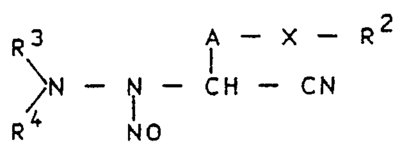
(II)

worin A, X, $R^2$, $R^3$ und $R^4$ die in einem oder mehreren der Ansprüche 1 bis 6 angegebenen Bedeutungen besitzen, zu einer Verbindung der allgemeinen Formel Ia

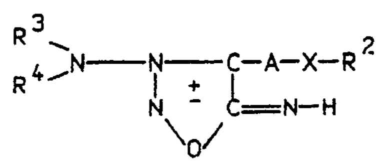
(Ia)

oder einem Säureadditionssalz davon cyclisiert wird und diese oder ein Säureadditionssalz davon für den Fall, daß eine Verbindung der Formel I mit $R^1$ = $-COR^5$ hergestellt wird, mit einem Acylierungsmittel acyliert wird, das den Rest $-COR^5$ einführt, und die erhaltene Verbindung gegebenenfalls in ein Säureadditionssalz überführt wird.
8. Verfahren nach Anspruch 7, dadurch gekennzeichnet, daß die Cyclisierung in einem Lösungs- oder Dispergiermittel bei Temperaturen von 0 bis 40°C, vorzugsweise 0 bis 20°C, mit Hilfe eines Cyclisierungsmittels, das in wäßriger Lösung einen pH-Wert von 3 oder darunter einstellt, durchgeführt wird.
9. Substituierte N-Nitroso-amino-acetonitrile der allgemeinen Formel II

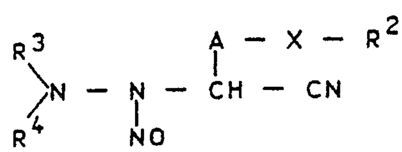
(II)

und ihre pharmakologisch annehmbaren Säureadditionssalze, wobei
A eine geradkettige oder verzweigte Alkylenkette mit 1 bis 6 C-Atomen;
X eine der Gruppen -O- oder -S- ;
$R^2$ eine Alkylgruppe mit 1 bis 6 C-Atomen, eine Alkenyloder Alkinylgruppe mit jeweils 3 bis 6 C-Atomen, eine gegebenenfalls substituierte Aralkylgruppe mit 1 bis 4 C-Atomen in der Alkyl- und 6 bis 10 C-Atomen in der Arylgruppe oder eine gegebenenfalls substituierte Arylgruppe mit 6 bis 10 C-Atomen;
$R^3$ und $R^4$, die gleich oder verschieden sein können, Alkylgruppen mit 1 bis 6 C-Atomen, Alkenyl-, Alkinyl- oder Cycloalkylgruppen mit jeweils 3 bis 6 C-Atomen, gegebenenfalls substituierte Aralkylgruppen mit 1 bis 4 C-Atomen in der Alkylgruppe und 6 bis 10 C-Atomen in der Arylgruppe, gegebenenfalls substituierte Arylgruppen mit 6 bis 10 C-Atomen, heterocyclische Gruppen mit 3 bis 5 Ring-C-Atomen und der Gruppe $-S(O)_m-$ oder $R^3$ und $R^4$ zusammen mit dem Stickstoffatom, an das sie gebunden sind, einen heterocyclischen Ring der Formel

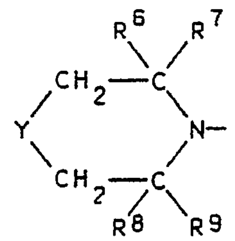 ;

$R^5$ einen aliphatischen Rest mit 1 bis 6 C-Atomen, der auch durch Alkoxy mit 1 bis 6 C-Atomen oder durch einen aliphatischen Thiorest mit bis zu 4 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen, der auch durch Alkoxy mit 1 bis 6 C-Atomen substituiert sein kann; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 4 C-Atomen und/oder 1 bis 3 Alkoxyreste mit 1 bis 3 C-Atomen und/oder 1 oder 2 Nitrogruppen und/oder 1 oder 2 Hydroxygruppen und/oder 1 oder 2 Alkylcarbonyloxyreste mit 1 bis 4 C-Atomen und/oder 1 bis 3 Alkylthioreste mit 1 bis 4-C-Atomen und/oder einen Trifluormethylrest und/oder einen Imidazolylrest mono-, di- oder trisubstituierten Arylrest mit 6 bis 10 C-Atomen; Imidazolyl; Pyridyl; Thienyl; Styryl;
m eine der Zahlen 0, 1 oder 2;
Y eine der Gruppen $-(CH_2)_n-$, -O-, $-S(O)_n-$ oder $-N(R^{10})-$;
$R^6$, $R^7$, $R^8$ und $R^9$, die gleich oder verschieden sein können, Wasserstoff oder Alkylgruppen mit 1 bis 4 C-Atomen;
n eine der Zahlen 0, 1 oder 2;
$R^{10}$ einen Alkylrest mit 1 bis 4 C-Atomen, einen gegebenenfalls substituierten Arylrest mit 6 bis 12 C-Atomen oder einen Rest der Formel $-COOR^{11}$, -COH, $-COR^{11}$ oder $-S(O)_2R^{11}$;
$R^{11}$ einen Alkylrest mit 1 bis 4 C-Atomen
bedeuten.

10. Verfahren zur Herstellung der in Anspruch 9 angegebenen Verbindungen der Formel II, dadurch gekennzeichnet, daß

a) eine Verbindung der Formel IV

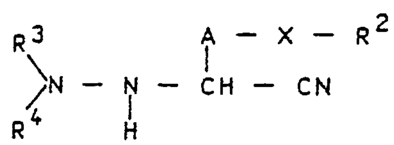 (IV)

worin A, X, $R^2$, $R^3$ und $R^4$ die in Anspruch 9 angegebene Bedeutung besitzen, nitrosiert wird, oder daß b) ein Säureadditionssalz eines substituierten 3-Aminosydnonimins der Formel Ia

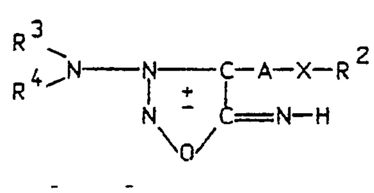 (Ia)

worin A, X, $R^2$, $R^3$ und $R^4$ die in Anspruch 9 angegebene Bedeutung besitzen mit einer Base umgesetzt wird.

11. Verwendung von substituierten 3-Amino-sydnoniminen nach einem oder mehreren der Ansprüche 1 bis 6 oder ihrer pharmakologisch annehmbaren Säureadditionssalze und/oder der substituierten N-Nitroso-aminoacetonitrile des Anspruchs 9 oder ihrer pharmakologisch annehmbaren Säureadditionssalze als pharmakologische Wirkstoffe zur Herstellung pharmazeutischer Präparate.

12. Pharmazeutisches Präparat, dadurch gekennzeichnet, daß es ein substituiertes 3-Aminosydnonimin nach einem oder mehreren der Ansprüche 1 bis 6 und/oder ein pharmakologisch annehmbares Säureadditionssalz davon und/oder ein substituiertes N-Nitroso-aminoacetonitril des Anspruchs 9 oder ein pharmakologisch annehmbares Säureadditionssalz davon oder mehrere derartiger Verbindungen als Wirkstoff oder Wirkstoffe zusammen mit einem oder mehreren pharmazeutisch annehmbaren Träger-und/oder Zusatzstoffen und gegebenenfalls noch eine oder mehrere andere pharmakologische Wirkstoffe enthält.

Patentansprüche für folgenden Vertragsstaat: ES

1. Verfahren zur Herstellung substituierter 3-Aminosydnonimine der allgemeinen Formel I

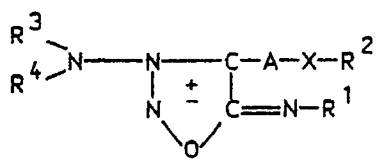

(I)

und ihrer pharmakologisch annehmbaren Säureadditionssalze,
worin
A eine geradkettige oder verzweigte Alkylenkette mit 1 bis 6 C-Atomen;
X eine der Gruppen -O- oder -S- ;
$R^1$ Wasserstoff oder den Rest $-COR^5$;
$R^2$ eine Alkylgruppe mit 1 bis 6 C-Atomen, eine Alkenyl-oder Alkinylgruppe mit jeweils 3 bis 6 C-Atomen, eine gegebenenfalls substituierte Aralkylgruppe mit 1 bis 4 C-Atomen in der Alkyl- und 6 bis 10 C-Atomen in der Arylgruppe oder eine gegebenenfalls substituierte Arylgruppe mit 6 bis 10 C-Atomen;
$R^3$ und $R^4$, die gleich oder verschieden sein können, Alkylgruppen mit 1 bis 6 C-Atomen, Alkenyl-, Alkinyl- oder Cycloalkylgruppen mit jeweils 3 bis 6 C-Atomen, gegebenenfalls substituierte Aralkylgruppen mit 1 bis 4 C-Atomen in der Alkylgruppe und 6 bis 10 C-Atomen in der Arylgruppe, gegebenenfalls substituierte Arylgruppen mit 6 bis 10 C-Atomen, heterocyclische Gruppen mit 3 bis 5 Ring-C-Atomen und der Gruppe $-S(O)_m-$ oder $R^3$ und $R^4$ zusammen mit dem Stickstoffatom, an das sie gebunden sind, einen heterocyclischen Ring der Formel

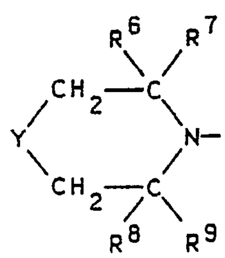

;

$R^5$ einen aliphatischen Rest mit 1 bis 6 C-Atomen, der auch durch Alkoxy mit 1 bis 6 C-Atomen oder durch einen aliphatischen Thiorest mit bis zu 4 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen, der auch durch Alkoxy mit 1 bis 6 C-Atomen substituiert sein kann; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 4 C-Atomen und/oder 1 bis 3 Alkoxyreste mit 1 bis 3 C-Atomen und/oder 1 oder 2 Nitrogruppen und/oder 1 oder 2 Hydroxygruppen und/oder 1 oder 2 Alkylcarbonyloxyreste mit 1 bis 4 C-Atomen und/oder 1 bis 3 Alkylthioreste mit 1 bis 4-C-Atomen und/oder einen Trifluormethylrest und/oder einen Imidazolylrest mono-, di- oder trisubstituierten Arylrest mit 6 bis 10 C-Atomen; Imidazolyl; Pyridyl; Thienyl; Styryl;
m eine der Zahlen 0, 1 oder 2;
Y eine der Gruppen $-(CH_2)_n-$, -O-, $-S(O)_n-$ oder $-N(R^{10})-$;
$R^6$, $R^7$, $R^8$ und $R^9$, die gleich oder verschieden sein können, Wasserstoff oder Alkylgruppen mit 1 bis 4 C-Atomen;
n eine der Zahlen 0, 1 oder 2;
$R^{10}$ einen Alkylrest mit 1 bis 4 C-Atomen, einen gegebe nenfalls substituierten Arylrest mit 6 bis 12 C-Atomen oder einen Rest der Formel $-COOR^{11}$, -COH, $-COR^{11}$ oder $-S(O)_2R^{11}$;
$R^{11}$ einen Alkylrest mit 1 bis 4 C-Atomen

bedeuten, dadurch gekennzeichnet, daß eine Verbindung der allgemeinen Formel II

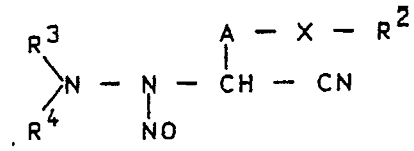

(II)

worin A, X, $R^2$, $R^3$ und $R^4$ die angegebenen Bedeutungen besitzen, zu einer Verbindung der allgemeinen Formel Ia

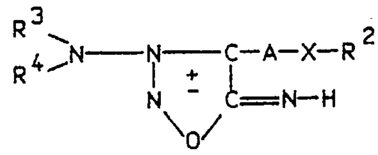

(Ia)

oder einem Säureadditionssalz davon cyclisiert wird und diese oder ein Säureadditionssalz davon für den Fall, daß eine Verbindung der Formel I mit $R^1$ = $-COR^5$ hergestellt wird, mit einem Acylierungsmittel acyliert wird, das den Rest $-COR^5$ einführt, und die erhaltene Verbindung gegebenenfalls in ein Säureadditionssalz überführt wird.

2. Verfahren nach Anspruch 1, dadurch gekennzeichnet, daß die Cyclisierung in einem Lösungs- oder Dispergiermittel bei Temperaturen von 0 bis 40°C, vorzugsweise 0 bis 20°C, mit Hilfe eines Cyclisierungsmittels, das in wäßriger Lösung einen pH-Wert von 3 oder darunter einstellt, durchgeführt wird.

3. Verfahren nach Anspruch 1 und/oder 2, dadurch gekennzeichnet, daß eine Ausgangsverbindung der Formel II eingesetzt wird, bei der $R^3$ und $R^4$ zusammen mit dem Stickstoffatom, an das sie gebunden sind, einen heterocyclischen Ring der Formel

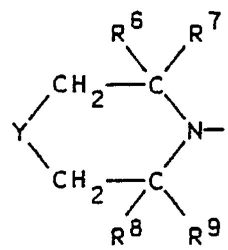

bedeuten, wobei Y vorzugsweise eine der Gruppen $-(CH_2)_0-$, $-(CH_2)_1-$, -O-, -S- oder $-S(O)_2-$bedeutet, und/oder $R^6$, $R^7$, $R^8$ und $R^9$, die gleich oder verschieden sein können, Wasserstoffatome oder Methylgruppen bedeuten.

4. Verfahren nach einem oder mehreren der Ansprüche 1 bis 3, dadurch gekennzeichnet, daß eine Ausgangsverbindung der Formel II eingesetzt wird, bei der $R^2$ einen Alkylest mit 1 bis 6 C-Atomen, einen Alkenyl-oder Alkinylest mit jeweils 3 bis 5 C-Atomen, einen Aralkylrest mit 1 bis 3 C-Atomen in der Alkylgruppe und einem unsubstituierten, mono- oder disubstituierten Phenylrest als Arylgruppe oder einen unsubstituierten, mono- oder disubstituierten Phenylrest bedeutet.

5. Verfahren nach einem oder mehreren der Ansprüche 1 bis 4, dadurch gekennzeichnet, daß eine Ausgangsverbindung der Formel II eingesetzt wird, bei der $R^2$ einen Alkylrest mit 1 bis 4 C-Atomen, einen Alkenylrest mit 3 bis 5 C-Atomen, einen Phenalkylrest mit 1 bis 3 C-Atomen in der Alkylgruppe oder einen Phenylrest bedeutet.

6. Verfahren nach einem oder mehreren der Ansprüche 1 bis 5, dadurch gekennzeichnet, daß eine Ausgangsverbindung der Formel II eingesetzt wird, bei der
$R^2$ Ethyl, Phenyl, Benzyl oder Allyl,
$R^3$ und $R^4$ zusammen mit dem Stickstoffatom, an das sie gebunden sind, Morpholino oder 2,6-Dimethylpiperidino,
$R^5$ Ethoxy, Phenyl oder Methoxyphenyl und
A Methylen oder 1-Methyl-ethylen
bedeuten.

7. Verfahren nach einem oder mehreren der Ansprüche 1 bis 6, dadurch gekennzeichnet, daß die Ausgangsverbindung so ausgewählt wird, daß 4-Benzylthiomethyl-3-(2,6-dimethylpiperidino)sydnonimin und seine pharmakologisch annehmbaren Säureadditionssalze, insbesondere sein Hydrochlorid entsteht.

8. Verfahren nach einem oder mehreren der Ansprüche 1 bis 6, dadurch gekennzeichnet, daß die Ausgangsverbindung so ausgewählt wird, daß 3-(2,6-Dimethylpiperidino)-4-phenoxymethylsydnonimin und seine pharmakologisch annehmbaren Säureadditionssalze, insbesondere sein Hydrochlorid entsteht.

9. Verfahren zur Herstellung substituierter N-Nitroso-amino-acetonitrile der allgemeinen Formel II

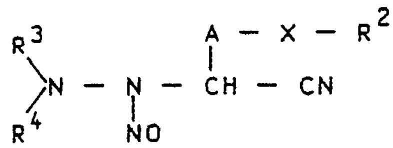

(II)

und ihrer pharmakologisch annehmbaren Säureadditionssalze, wobei
A eine geradkettige oder verzweigte Alkylenkette mit 1 bis 6 C-Atomen;
X eine der Gruppen -O- oder -S- ;
$R^2$ eine Alkylgruppe mit 1 bis 6 C-Atomen, eine Alkenyl oder Alkinylgruppe mit jeweils 3 bis 6 C-Atomen, eine gegebenenfalls substituierte Aralkylgruppe mit 1 bis 4 C-Atomen in der Alkyl- und 6 bis 10 C-Atomen in der Arylgruppe oder eine gegebenenfalls substituierte Arylgruppe mit 6 bis 10 C-Atomen;
$R^3$ und $R^4$, die gleich oder verschieden sein können, Alkylgruppen mit 1 bis 6 C-Atomen, Alkenyl-, Alkinyl- oder Cycloalkylgruppen mit jeweils 3 bis 6 C-Atomen, gegebenenfalls substituierte Aralkylgruppen mit 1 bis 4 C-Atomen in der Alkylgruppe und 6 bis 10 C-Atomen in der Arylgruppe, gegebenenfalls substituierte Arylgruppen mit 6 bis 10 C-Atomen, heterocyclische Gruppen mit 3 bis 5 Ring-C-Atomen und der Gruppe $-S(O)_m-$ oder $R^3$ und $R^4$ zusammen mit dem Stickstoffatom, an das sie gebunden sind, einen heterocyclischen Ring der Formel

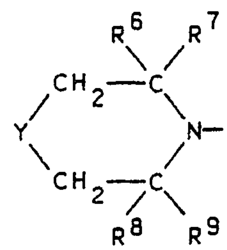

;

$R^5$ einen aliphatischen Rest mit 1 bis 6 C-Atomen, der auch durch Alkoxy mit 1 bis 6 C-Atomen oder durch einen aliphatischen Thiorest mit bis zu 4 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen, der auch durch Alkoxy mit 1 bis 6 C-Atomen substituiert sein kann; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogen atome und/oder 1 bis 3 Alkylreste mit 1 bis 4 C-Atomen und/oder 1 bis 3 Alkoxyreste mit 1 bis 3 C-Atomen und/oder 1 oder 2 Nitrogruppen und/oder 1 oder 2 Hydroxygruppen und/oder 1 oder 2 Alkylcarbonyloxyreste mit 1 bis 4 C-Atomen und/oder 1 bis 3 Alkylthioreste mit 1 bis 4-C-Atomen und/oder einen Trifluormethylrest und/oder einen Imidazolylrest mono-, di- oder trisubstituierten Arylrest mit 6 bis 10 C-Atomen; Imidazolyl; Pyridyl; Thienyl; Styryl;
m eine der Zahlen 0, 1 oder 2;
Y eine der Gruppen $-(CH_2)_n-$, -O-, $-S(O)_n-$ oder $-N(R^{10})-$;
$R^6$, $R^7$, $R^8$ und $R^9$, die gleich oder verschieden sein können, Wasserstoff oder Alkylgruppen mit 1 bis 4 C-Atomen; n eine der Zahlen 0, 1 oder 2;
$R^{10}$ einen Alkylrest mit 1 bis 4 C-Atomen, einen gegebenenfalls substituierten Arylrest mit 6 bis 12 C-Atomen oder einen Rest der Formel $-COOR^{11}$, -COH, $-COR^{11}$ oder $-S(O)_2R^{11}$;
$R^{11}$ einen Alkylrest mit 1 bis 4 C-Atomen bedeuten, dadurch gekennzeichnet, daß
a) eine Verbindung der Formel IV

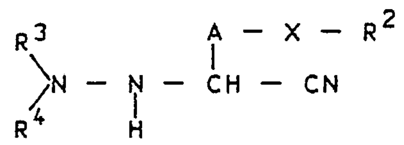

(IV)

worin A, X, $R^2$, $R^3$ und $R^4$ die angegebene Bedeutung besitzen, nitrosiert wird, oder daß b) ein Säureadditionssalz eines substituierten 3-Aminosydnonimins der Formel Ia

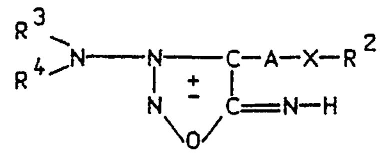

(Ia)

worin A, X, $R^2$, $R^3$ und $R^4$ die angegebene Bedeutung besitzen. mit einer Base umgesetzt wird.

10. Verwendung von substituierten 3-Aminosydnoniminen der allgemeinen Formel I

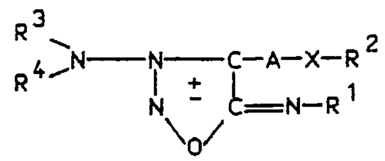

(I)

und ihrer pharmakologisch annehmbaren Säureadditionssalze,
worin
A eine geradkettige oder verzweigte Alkylenkette mit 1 bis 6 C-Atomen;
X eine der Gruppen -O- oder -S- ;
$R^1$ Wasserstoff oder den Rest $-COR^5$;
$R^2$ eine Alkylgruppe mit 1 bis 6 C-Atomen, eine Alkenyl-oder Alkinylgruppe mit jeweils 3 bis 6 C-Atomen, eine gegebenenfalls substituierte Aralkylgruppe mit 1 bis 4 C-Atomen in der Alkyl- und 6 bis 10 C-Atomen in der Arylgruppe oder eine gegebenenfalls substituierte Arylgruppe mit 6 bis 10 C-Atomen;
$R^3$ und $R^4$, die gleich oder verschieden sein können, Alkylgruppen mit 1 bis 6 C-Atomen, Alkenyl-, Alkinyl- oder Cycloalkylgruppen mit jeweils 3 bis 6 C-Atomen, gegebenenfalls substituierte Aralkylgruppen mit 1 bis 4 C-Atomen in der Alkylgruppe und 6 bis 10 C-Atomen in der Arylgruppe, gegebenenfalls substituierte Arylgruppen mit 6 bis 10 C-Atomen, heterocyclische Gruppen mit 3 bis 5 Ring-C-Atomen und der Gruppe $-S(O)_m$ oder $R^3$ und $R^4$ zusammen mit dem Stickstoffatom, an das sie gebunden sind, einen heterocyclischen Ring der Formel

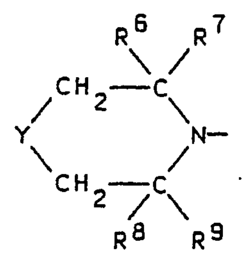

;

$R^5$ einen aliphatischen Rest mit 1 bis 6 C-Atomen, der auch durch Alkoxy mit 1 bis 6 C-Atomen oder durch einen aliphatischen Thiorest mit bis zu 4 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen, der auch durch Alkoxy mit 1 bis 6 C-Atomen substituiert sein kann; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit bis 4 C-Atomen und/oder 1 bis 3 Alkoxyreste mit 1 bis 3 C-Atomen und/oder 1 oder 2 Nitrogruppen und/oder 1 oder 2 Hydroxygruppen und/oder 1 oder 2 Alkylcarbonyloxyreste mit 1

bis 4 C-Atomen und/oder 1 bis 3 Alkylthioreste mit 1 bis 4-C-Atomen und/oder einen Trifluormethylrest und/oder einen Imidazolylrest mono-, di- oder trisubstituierten Arylrest mit 6 bis 10 C-Atomen; Imidazolyl; Pyridyl; Thienyl; Styryl;
m eine der Zahlen 0, 1 oder 2;
Y eine der Gruppen $-(CH_2)_n-$, -O-, $-S(O)_n-$ oder $-N(R^{10})-$;
$R^6$, $R^7$, $R^8$ und $R^9$, die gleich oder verschieden sein können, Wasserstoff oder Alkylgruppen mit 1 bis 4 C-Atomen; n eine der Zahlen 0, 1 oder 2;
$R^{10}$ einen Alkylrest mit 1 bis 4 C-Atomen, einen gegebe nenfalls substituierten Arylrest mit 6 bis 12 C-Atomen oder einen Rest der Formel $-COOR^{11}$, -COH, $-COR^{11}$ oder $-S(O)_2R^{11}$;
$R^{11}$ einen Alkylrest mit 1 bis 4 C-Atomen bedeuten und/oder von substituierten N-Nitroso-amino-acetonitrilen der allgemeinen Formel II

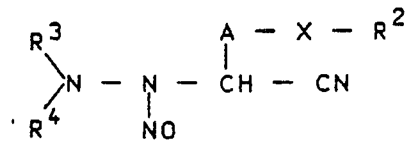

(II)

und ihrer pharmakologisch annehmbaren Säureadditionssalze, wobei
A eine geradkettige oder verzweigte Alkylenkette mit 1 bis 6 C-Atomen;
X eine der Gruppen -O- oder -S- ;
$R^2$ eine Alkylgruppe mit 1 bis 6 C-Atomen, eine Alkenyl-oder Alkinylgruppe mit jeweils 3 bis 6 C-Atomen, eine gegebenenfalls substituierte Aralkylgruppe mit 1 bis 4 C-Atomen in der Alkyl- und 6 bis 10 C-Atomen in der Arylgruppe oder eine gegebenenfalls substituierte Arylgruppe mit 6 bis 10 C-Atomen;
$R^3$ und $R^4$, die gleich oder verschieden sein können, Alkylgruppen mit 1 bis 6 C-Atomen, Alkenyl-, Alkinyl- oder Cycloalkylgruppen mit jeweils 3 bis 6 C-Atomen, gegebenenfalls substituierte Aralkylgruppen mit 1 bis 4 C-Atomen in der Alkylgruppe und 6 bis 10 C-Atomen in der Arylgruppe, gegebenenfalls substituierte Arylgruppen mit 6 bis 10 C-Atomen, heterocyclische Gruppen mit 3 bis 5 Ring-C-Atomen und der Gruppe $-S(O)_m-$ oder $R^3$ und $R^4$ zusammen mit dem Stickstoffatom, an das sie gebunden sind, einen heterocyclischen Ring der Formel

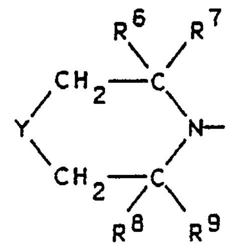

;

$R^5$ einen aliphatischen Rest mit 1 bis 6 C-Atomen, der auch durch Alkoxy mit 1 bis 6 C-Atomen oder durch einen aliphatischen Thiorest mit bis zu 4 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen, der auch durch Alkoxy mit 1 bis 6 C-Atomen substituiert sein kann; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 4 C-Atomen und/oder 1 bis 3 Alkoxyreste mit 1 bis 3 C-Atomen und/oder 1 oder 2 Nitrogruppen und/oder 1 oder 2 Hydroxygruppen und/oder 1 oder 2 Alkylcarbonyloxyreste mit 1 bis 4 C-Atomen und/oder 1 bis 3 Alkylthioreste mit 1 bis 4-C-Atomen und/oder einen Trifluormethylrest und/oder einen Imidazolylrest mono-, di- oder trisubstituierten Arylrest mit 6 bis 10 C-Atomen; Imidazolyl; Pyridyl; Thienyl; Styryl;
m eine der Zahlen 0, 1 oder 2;
Y eine der Gruppen $-(CH_2)_n-$, -O-, $-S(O)_n-$ oder $-N(R^{10})-$;
$R^6$, $R^7$, $R^8$ und $R^9$, die gleich oder verschieden sein können, Wasserstoff oder Alkylgruppen mit 1 bis 4 C-Atomen; n eine der Zahlen 0, 1 oder 2;
$R^{10}$ einen Alkylrest mit 1 bis 4 C-Atomen, einen gegebe nenfalls substituierten Arylrest mit 6 bis 12 C-Atomen oder einen Rest der Formel $-COOR^{11}$, -COH, $-COR^{11}$ oder $-S(O)_2R^{11}$;
$R^{11}$ einen Alkylrest mit 1 bis 4 C-Atomen

bedeuten als pharmakologische Wirkstoffe zur Herstellung pharmazeutischer Präparate.

11. Verfahren zur Herstellung eines pharmazeutischen Präparats, dadurch gekennzeichnet, daß ein substituiertes 3-Aminosydnonimin der allgemeinen Formel I

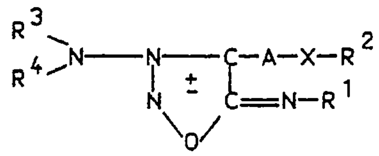

(I)

und/oder ein pharmakologisch annehmbares Säureadditionssalz davon, worin

A eine geradkettige oder verzweigte Alkylenkette mit 1 bis 6 C-Atomen;

X eine der Gruppen -O- oder -S- ;

$R^1$ Wasserstoff oder den Rest $-COR^5$;

$R^2$ eine Alkylgruppe mit 1 bis 6 C-Atomen, eine Alkenyl-oder Alkinylgruppe mit jeweils 3 bis 6 C-Atomen, eine gegebenenfalls substituierte Aralkylgruppe mit 1 bis 4 C-Atomen in der Alkyl- und 6 bis 10 C-Atomen in der Arylgruppe oder eine gegebenenfalls substituierte Arylgruppe mit 6 bis 10 C-Atomen;

$R^3$ und $R^4$, die gleich oder verschieden sein können, Alkylgruppen mit 1 bis 6 C-Atomen, Alkenyl-, Alkinyl- oder Cycloalkylgruppen mit jeweils 3 bis 6 C-Atomen, gegebenenfalls substituierte Aralkylgruppen mit 1 bis 4 C-Atomen in der Alkylgruppe und 6 bis 10 C-Atomen in der Arylgruppe, gegebenenfalls substituierte Arylgruppen mit 6 bis 10 C-Atomen, heterocyclische Gruppen mit 3 bis 5 Ring-C-Atomen und der Gruppe $-S(O)_m-$ oder $R^3$ und $R^4$ zusammen mit dem Stickstoffatom, an das sie gebunden sind, einen heterocyclischen Ring der Formel

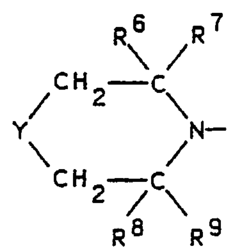

$R^5$ einen aliphatischen Rest mit 1 bis 6 C-Atomen, der auch durch Alkoxy mit 1 bis 6 C-Atomen oder durch einen aliphatischen Thiorest mit bis zu 4 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen, der auch durch Alkoxy mit 1 bis 6 C-Atomen substituiert sein kann; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 4 C-Atomen und/oder 1 bis 3 Alkoxyreste mit 1 bis 3 C-Atomen und/oder 1 oder 2 Nitrogruppen und/oder 1 oder 2 Hydroxygruppen und/oder 1 oder 2 Alkylcarbonyloxyreste mit 1 bis 4 C-Atomen und/oder 1 bis 3 Alkylthioreste mit 1 bis 4-C-Atomen und/oder einen Trifluormethylrest und/oder einen Imidazolylrest mono-, di- oder trisubstituierten Arylrest mit 6 bis 10 C-Atomen; Imidazolyl; Pyridyl; Thienyl; Styryl;

m eine der Zahlen 0, 1 oder 2;

Y eine der Gruppen $-(CH_2)_n-$, -O-, $-S(O)_n$ oder $-N(R^{10})-$;

$R^6$, $R^7$, $R^8$ und $R^9$, die gleich oder verschieden sein kön nen, Wasserstoff oder Alkylgruppen mit 1 bis 4 C-Atomen; n eine der Zahlen 0, 1 oder 2;

$R^{10}$ einen Alkylrest mit 1 bis 4 C-Atomen, einen gegebenenfalls substituierten Arylrest mit 6 bis 12 C-Atomen oder einen Rest der Formel $-COOR^{11}$, -COH, $-COR^{11}$ oder $-S(O)_2R^{11}$;

$R^{11}$ einen Alkylrest mit 1 bis 4 C-Atomen bedeuten, und/oder ein substituiertes N-Nitroso-aminoacetonitril der allgemeinen Formel II

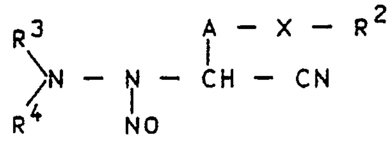

(II)

und/oder ein pharmakologisch annehmbaren Säureadditionssalz davon, wobei
A eine geradkettige oder verzweigte Alkylenkette mit 1 bis 6 C-Atomen;
X eine der Gruppen -O- oder -S- ;
$R^2$ eine Alkylgruppe mit 1 bis 6 C-Atomen, eine Alkenyl-oder Alkinylgruppe mit jeweils 3 bis 6 C-Atomen, eine gegebenenfalls substituierte Aralkylgruppe mit 1 bis 4 C-Atomen in der Alkyl- und 6 bis 10 C-Atomen in der Arylgruppe oder eine gegebenenfalls substituierte Arylgruppe mit 6 bis 10 C-Atomen;
$R^3$ und $R^4$, die gleich oder verschieden sein können, Alkylgruppen mit 1 bis 6 C-Atomen, Alkenyl-, Alkinyl- oder Cycloalkylgruppen mit jeweils 3 bis 6 C-Atomen, gegebenenfalls substituierte Aralkylgruppen mit 1 bis 4 C-Atomen in der Alkylgruppe und 6 bis 10 C-Atomen in der Arylgruppe, gegebenenfalls substituierte Arylgruppen mit 6 bis 10 C-Atomen, heterocyclische Gruppen mit 3 bis 5 Ring-C-Atomen und der Gruppe $-S(O)_m-$ oder $R^3$ und $R^4$ zusammen mit dem Stickstoffatom, an das sie gebunden sind, einen heterocyclischen Ring der Formel

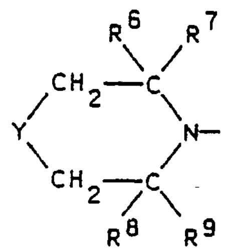

;

$R^5$ einen aliphatischen Rest mit 1 bis 6 C-Atomen, der auch durch Alkoxy mit 1 bis 6 C-Atomen oder durch einen aliphatischen Thiorest mit bis zu 4 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen, der auch durch Alkoxy mit 1 bis 6 C-Atomen substituiert sein kann; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 4 C-Atomen und/oder 1 bis 3 Alkoxyreste mit 1 bis 3 C-Atomen und/oder 1 oder 2 Nitrogruppen und/oder 1 oder 2 Hydroxygruppen und/oder 1 oder 2 Alkylcarbonyloxyreste mit 1 bis 4 C-Atomen und/oder 1 bis 3 Alkylthioreste mit 1 bis 4-C-Atomen und/oder einen Trifluormethylrest und/oder einen Imidazolylrest mono-, di- oder trisubstituierten Arylrest mit 6 bis 10 C-Atomen; Imidazolyl; Pyridyl; Thienyl; Styryl;
m eine der Zahlen 0, 1 oder 2;
Y eine der Gruppen $-(CH_2)_n-$, -O-, $-S(O)_n-$ oder $-N(R^{10})-$;
$R^6$, $R^7$, $R^8$ und $R^9$, die gleich oder verschieden sein können, Wasserstoff oder Alkylgruppen mit 1 bis 4 C-Atomen;
n eine der Zahlen 0, 1 oder 2;
$R^{10}$ einen Alkylrest mit 1 bis 4 C-Atomen, einen gegebe nenfalls substituierten Arylrest mit 6 bis 12 C-Atomen oder einen Rest der Formel $-COOR^{11}$, -COH, $-COR^{11}$ oder $-S(O)_2R^{11}$;
$R^{11}$ einen Alkylrest mit 1 bis 4 C-Atomen
bedeuten, oder mehrere derartiger Verbindungen als Wirkstoff oder Wirkstoffe zusammen mit einem oder mehreren pharmazeutisch annehmbaren Träger- und/oder Zusatzstoffen und gegebenenfaljs noch einem oder mehreren anderen pharmakologischen Wirktoffen in an sich bekannter Weise in eine zur Verabreichung geeignete pharmazeutische Zubereitung überführt werden.

Europäisches Patentamt

# EUROPÄISCHER RECHERCHENBERICHT

Nummer der Anmeldung

**EP 90 11 2031**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 312 773 (CASSELLA AG)<br>* Ansprüche 1,7-10 * | 1,7,8,11,<br>12 | C 07 D 271/04<br>C 07 D 413/04<br>C 07 D 295/22<br>C 07 D 211/98<br>C 07 D 265/30<br>C 07 C 291/08<br>A 61 K 31/41 //<br>(C 07 D 413/04<br>C 07 D 271:00<br>C 07 D 211:00 )<br>(C 07 D 413/04<br>C 07 D 271:00<br>C 07 D 265:00 ) |
| A | EP-A-0 023 343 (CASSELLA AG)<br>* Ansprüche 1,11,13-16 * | 1,7,9-12 | |
| A,D | DE-A-1 695 897 (TAKEDA CHEMICAL INDUSTRIES, LTD.)<br>* Ansprüche 1-3,6 * | 1,7,11,12 | |
| A | DE-A-1 670 127 (C.H. BOEHRINGER SOHN)<br>* Ansprüche 1,3,8 * | 1,7,11,12 | |
| A | EP-A-0 210 474 (CASSELLA AG)<br>* Ansprüche 1,9,10 * | 1,11,12 | |
| A,P | EP-A-0 324 408 (CASSELLA AG)<br>* Ansprüche 1,6-8 * | 9-12 | |
| A | DE-A-2 131 826 (SANDOZ AG)<br>* Ansprüche 1-3; Seiten 1-6 * | 9-12 | RECHERCHIERTE SACHGEBIETE (Int. Cl.5) |
| A | DE-A-2 126 035 (SANDOZ AG)<br>* Seite 4, Zeile 9 - Seite 8, Zeile 12 * | 9,11,12 | C 07 C 291/00<br>C 07 D 211/00<br>C 07 D 265/00<br>C 07 D 271/00<br>C 07 D 295/00<br>C 07 D 413/00 |

Der vorliegende Recherchenbericht wurde für alle Patentansprüche erstellt

| Recherchenort | Abschlußdatum der Recherche | Prüfer |
|---|---|---|
| Berlin | 12 Oktober 90 | HASS C V F |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument